# EUROPEAN PATENT SPECIFICATION

(11) **EP 3 168 215 B1**
(45) Date of publication and mention of the grant of the patent: **20.05.2020**
(21) Application number: 16198078.4
(22) Date of filing: 10.11.2016
(51) Int. Cl.: C07D 401/12, C07D 211/82

(54) **PROCESS FOR PREPARATION OF BARNIDIPINE**
VERFAHREN ZUR HERSTELLUNG VON BARNIDIPIN
PROCÉDÉ DE PRÉPARATION DE BARNIDIPINE

(30) Priority: 10.11.2015 IN 4298MU2015
(43) Date of publication of application: 17.05.2017
(73) Proprietor: USV Private Limited, 400 088 Mumbai (IN)
(72) Inventor: Patkar, Laxmikant Narhari, 400 088 Mumbai (IN); Damle, Subhash Vishwanath, 400 088 Mumbai (IN); Bhopalkar, Rajesh Ganpat, 400 088 Mumbai (IN); Gaware, Rawindra Pandharinath, 400 088 Mumbai (IN)
(74) Representative: Schön, Christoph

(56) References cited:
- IN-A- 1299C HE2 011
- US-A- 5 463 064
- DATABASE WPI Week 201644 Thomson Scientific, London, GB; AN 2016-28906S XP002767850, -& CN 105 541 797 A (ZHAO J) 4 May 2016 (2016-05-04)
- DATABASE WPI Week 201033 Thomson Scientific, London, GB; AN 2010-C31565 XP002767851, -& CN 101 643 469 A (WUHAN BAIKE PHARM DEV CO LTD) 10 February 2010 (2010-02-10)
- WANG LI ET AL: "Synthesis of Barnidipine Hydrochloride", HUAGONG SHIKAN - CHEMICAL INDUSTRY TIMES, vol. 21, no. 6, 2007, pages 27-29, XP009193650, ISSN: 1002-154X
- TAMAZAWA K ET AL: "Stereoselectivity of a potent calcium antagonist, 1-benzyl-3-pyrrolidinyl methyl 2,6-dimethyl-4-(m-nitrophenyl)-1,4-dihydro pyridine-3,5-dicarboxylate", JOURNAL OF MEDICINAL CHEMISTRY, vol. 29, no. 12, 1 December 1986 (1986-12-01), pages 2504-2511, XP000564487, ISSN: 0022-2623, DOI: 10.1021/JM00162A013

## Description

### Field of the invention:

The present invention relates to a process for preparation of Barnidipine or a pharmaceutically acceptable salt thereof. The present invention relates to chiral compounds represented by Formula III and the use of compounds represented by Formula II or Formula III in the preparation of Barnidipine or a pharmaceutically acceptable salt thereof. The present invention further relates to a process for preparation of chiral compounds represented by Formula II or Formula III.

### Background of the invention:

Barnidipine hydrochloride is a 1,4-dihydropyridine Ca²⁺ antagonist developed and marketed by Yamanouchi Pharmaceutical (now Astellas Pharma). Barnidipine hydrochloride is a calcium channel blocker possessing potent antihypertensive activity. This drug stereospecifically binds to the binding sites of [³H]nitrendipine and [³H](+)PN200-110 in cardiac, brain and vascular tissues with high affinity.

It is available under the trade name of Hypoca®, Cyress®, Libradin® and Vasextan®. It is indicated for mild to moderate essential hypertension. It is available as 10 mg modified release capsule containing 10 mg of Barnidipine hydrochloride equivalent to 9.3 mg of Barnidipine per capsule and as 20 mg modified release capsule containing 20 mg of Barnidipine hydrochloride equivalent to 18.6 mg of Barnidipine per capsule. The recommended starting dosage is 10 mg once daily, in the morning. It may be increased to 20 mg once daily, if necessary.

It is chemically known as (+)-(3'S,4S)-1-benzyl-3-pyrrolidinylmethyl 1,4-dihydro-2,6-dimethyl-4-(3-nitrophenyl)-3,5-pyridinedicarboxylate hydrochloride or (4S)-1,4-dihydro-2,6-dimethyl-4-(3-nitrophenyl)-3,5-pyridinedicarboxylic acid methyl (3S)-1-(phenylmethyl)-3-pyrrolidinyl ester hydrochloride represented by Formula (I) below,

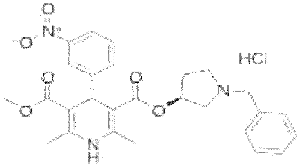

### Formula I

Barnidipine has two chiral centers, one in the dihydropyridine ring and one in the pyrrolidinyl ring. This compound has four optical isomers, (S, S), (R, R), (S, R) and (R, S). Barnidipine is the (S, S) isomer, which is the most potent and longest acting of the four isomers.

US5463064 discloses Barnidipine and process for preparation thereof. The process disclosed involves reacting (S)-1-benzyl-3-hydroxypyrrolidine with diketene to produce (S)-3-acetoacetoxy-1-benzylpyrrolidine followed by reaction with m-nitrobenzaldehyde and 3-aminocrotonic acid methyl ester to get a mixture of diastereomers. The process involves the use of column chromatography for separation of the mixture of diastereomers to obtain the desired isomer. The use of column chromatography makes the process ineffective at an industrial scale. This document further teaches the use of resolving agents for the separation of the desired isomers. The use of these techniques can result in low yield and high cost.

Journal of Medicinal Chemistry, 1986, 29, 2504 discloses a process similar to the process disclosed in US5463064. This document also teaches the use of resolving agents for separation of the desired isomer. The use of resolution technique can result in low yield and high cost

CN101643469B discloses the reaction of 2-cyanoethyl-3-oxobutanoate with m-nitrobenzaldehyde to get (2-cyanoethyl)-2-(3-nitrobenzylidene)-3-oxobutanoate followed by reaction with methyl-3-amino crotonate and hydrolysis of the resulting product to yield racemic 5-(methoxycarbonyl)-2,6-dimethyl-4-(3-nitrophenyl)-1,4-dihydropyridine-3-carboxylic acid. The racemic compound thus obtain is subjected to resolution using resolving agent such as cinchonine to obtain the R-isomer of 5-(methoxycarbonyl)-2,6-dimethyl-4-(3-nitrophenyl)-1,4-dihydropyridine-3-carboxylic acid, which is then converted to Barnidipine. The disclosed process involves the use of expensive resolving agents.

1299/CHE/2011 discloses the resolution of racemic 5-(methoxycarbonyl)-2,6-dimethyl-4-(3-nitrophenyl)-1,4-dihydropyridine-3-carboxylic acid using resolving agent such as L-norephedrine, D-norephedrine, L-proline, cinchonidine or quinidine to obtain the (R)-isomer of 5-(methoxycarbonyl)-2,6-dimethyl-4-(3-nitrophenyl)-1,4-dihydropyridine-3-carboxylic acid. The obtained compound is then coupled with (S)-1-benzyl-3-hydroxypyrrolidine or derivative thereof to obtain Barnidipine. This process involves the use of expensive resolving agents.

Wang Li et al.: "Synthesis of Barnidipine Hydrochloride", HUAGONG SHIKAN-CHEMICAL INDUSTRY TIMES, vol. 21, no. 6, 2007, pages 27-29 teach a preparation of Barnidipine hydrochloride via cyclocondensation from m-nitrobenzaldehyde, methyl acetoacetate and ammonium bicarbonate, hydrolysis, esterification with optically (S)-N-benzyl-3-pyrridinol, recrystallization and salification.

There exists a need to develop a simple, cost-effective and commercially viable process for the large scale preparation of Barnidipine or its pharmaceutically acceptable salt. The present invention provides an industrially viable and cost effective process for the large scale preparation of (R)-5-(methoxycarbonyl)-2,6-dimethyl-4-(3-nitrophenyl)-1,4-dihydropyridine-3-carboxylic acid, key intermediate in the preparation of Barnidipine or a pharmaceutically acceptable salt thereof. The present invention further provides chiral compounds represented by Formula II or Formula III and use of these compounds in the preparation of Barnidipine or a pharmaceutically acceptable salt thereof.

### Object of the invention:

An object of the present invention is to provide a simple, cost-effective and industrially viable process for the large scale preparation of Barnidipine or a pharmaceutically acceptable salt thereof.

Another object of the present invention is to provide chiral compounds represented by Formula II or Formula III and use of these compounds in the preparation of Barnidipine or a pharmaceutically acceptable salt thereof.

Yet another object of the present invention provides a cost effective process for the large scale preparation of (R)-5-(methoxycarbonyl)-2,6-dimethyl-4-(3-nitrophenyl)-1,4-dihydropyridine-3-carboxylic acid.

### Summary of the invention :

According to one aspect of the present invention, there is provided a process for preparation of Barnidipine or a pharmaceutically acceptable salt thereof, comprising the steps of,
a) providing a chiral compound of Formula II, 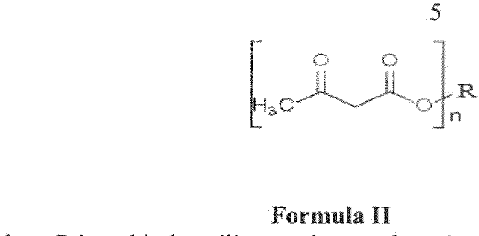 where R is a chiral auxiliary moiety; and n = 1 - 4;
b) treating said chiral compound of Formula II with m-nitrobenzaldehyde and methyl-3-aminocrotonate under Hantzsch reaction conditions to obtain a chiral compound of Formula III; 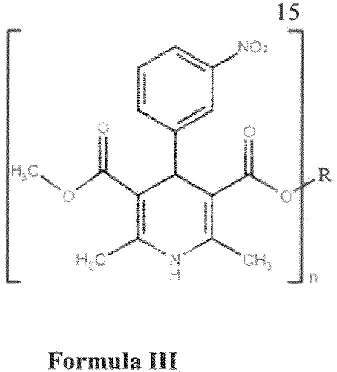 where R is a chiral auxiliary moiety; and n = 1 - 4; and
c) converting said chiral compound of Formula III to Barnidipine or a pharmaceutically acceptable salt thereof, wherein said chiral auxiliary moiety is selected from the group consisting of (2S,3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)- alpha substituted butanedioate. Preferably, n = I or 2.

Preferably, the chiral compound of Formula II is prepared by reacting a chiral auxiliary reactant with diketene, acetoacetic acid or derivative thereof.

Another aspect of the present invention provides a process, wherein the chiral auxiliary reactant is selected from 2S,3S-dihydroxybutanedioic acid, 2S,3S-dihydroxybutanedioic acid ester, (1R,2S,5R)-2-isopropyl-5-methyl cyclohexanol, N-benzyl-3(S)-hydroxy pyrrolidine, (S)-alpha hydroxy phenyl acetic acid, (S)-alpha hydroxy phenyl acetic acid ester, (S)-alpha hydroxy butanedioic acid or (S)-alpha hydroxy butanedioic acid ester and said derivative of acetoacetic acid is selected from acetoacetic acid ester or 2,2,6-trimethyl-4H-1,3-dioxin-4-one.

Another aspect of the present invention provides compound of Formula II selected from the group consisting of (2S,3S)-dimethyl 2,3-bis(3-oxobutanoyloxy)butanedioate, (2S,3S)-diethyl 2,3-bis(3-oxobutanoyloxy) butane dioate, (1R,2S,5R)-2-isopropyl-5-methyl-1-(3-oxobutanoyloxy) cyclohexane, (3S)-(acetoacetoxy)-1-benzylpyrrolidine, (S)-alpha (acetoacetoxy) phenyl acetic acid methyl ester, (S)-alpha (acetoacetoxy) phenyl acetic acid ethyl ester, Dimethyl-(S)-alpha(acetoacetoxy)-butanedioate and Diethyl-(S)-alpha (acetoacetoxy)-butane dioate.

Another aspect of the present invention provides compound of Formula III selected from the group consisting of, (2S,3S)-dimethyl-2,3-bis[(4S)-5-methoxycarbonyl-2,6-dimethyl-4-(3-nitrophenyl)- 1,4-dihydropyridine- 3-carboxy]butanedioate, (2S,3S)-diethyl-2,3-bis[(4S)-5-methoxycarbonyl-2,6-dimethyl-4-(3-nitrophenyl)-1,4-dihydropyridine-3-carboxy]butanedioate, (1R,2S,5R)-2-isopropyl-5-methyl-1-[(4S)-5-methoxycarbonyl-2,6-dimethyl- 4-(3-nitrophenyl)- 1,4-dihydropyridine-3-carboxy]cyclohexane, (3'S,4S)-1-benzyl-3-pyrrolidinyl- methyl-1,4-dihydro- 2,6-dimethyl-4-(3-nitrophenyl)-3,5-pyridinedicarboxylate, (S)-alpha[(4S)-5-methoxy carbonyl-2,6-dimethyl- 4-(3-nitrophenyl)- 1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha[(4S)-5-methoxycarbonyl- 2,6-dimethyl- 4-(3-nitrophenyl)-1,4-dihydropyridine-3-carboxy]butanedioate and Diethyl-(S)-alpha[(4S)-5-methoxy carbonyl-2,6-dimethyl- 4-(3-nitrophenyl)- 1,4-dihydropyridine-3-carboxy] butanedioate.

Preferably, Hantzsch reaction is carried out in the presence of a solvent selected from methanol, ethanol, n-propanol, isopropanol, n-butanol, toluene, acetonitrile, DMF, cyclohexane or ethylene glycol.

Another aspect of the present invention provides conversion of chiral compound of Formula III to Barnidipine or a pharmaceutically acceptable salt thereof comprising the steps of,
a) subjecting said chiral compound of Formula III to selective hydrolysis to obtain (R)-5-(methoxycarbonyl)-2,6-dimethyl-4-(3-nitrophenyl)-1,4-dihydro pyridine-3-carboxylic acid; and
b) converting said (R)-5-(methoxycarbonyl)-2,6-dimethyl-4-(3-nitro phenyl)-1,4-dihydropyridine-3-carboxylic acid to Barnidipine or a pharmaceutically acceptable salt thereof,
wherein said chiral compound of Formula III is selected from the group consisting of (2S,3S)-dimethyl- 2,3-bis[(4S)-5-methoxycarbonyl- 2,6-dimethyl-4-(3-nitrophenyl)-1,4-dihydropyridine-3-carboxy]butanedioate, (2S,3S)-diethyl-2,3-bis[(4S)-5-methoxycarbonyl-2,6-dimethyl-4-(3-nitrophenyl)-1,4-dihydropyridine-3-carboxy]butanedioate, (1R,2S,5R)-2-isopropyl-5-methyl- 1-[(4S)-5-methoxycarbonyl- 2,6-dimethyl- 4-(3-nitrophenyl)- 1,4-dihydropyridine-3-carboxy]cyclohexane, (S)-alpha[(4S)-5-methoxycarbonyl- 2,6-dimethyl- 4-(3-nitrophenyl)-1,4-dihydropyridine-3-carboxy] phenyl acetic acid methyl ester, (S)-alpha[(4S)- 5-methoxycarbonyl- 2,6-dimethyl- 4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy]butanedioate and Diethyl-(S)-alpha [(4S)-5-methoxycarbonyl-2,6-dimethyl-4-(3-nitrophenyl)-1,4-dihydropyridine-3-carboxy]butanedioate.

Preferably, selective hydrolysis in step a) is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxyethane, polyethylene glycol or water. More preferably, selective hydrolysis is carried out in the presence of ethylene glycol at a temperature of about 50° to 70° C.

Another aspect of the present invention provides a process for preparation of Barnidipine or a pharmaceutically acceptable salt thereof comprising the steps of,
a) providing a chiral compound of Formula III, 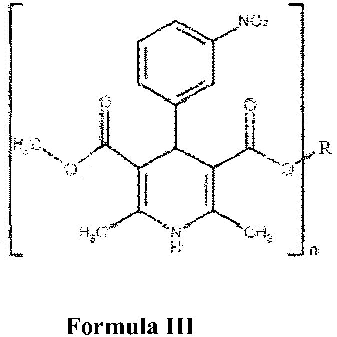 where R is a chiral auxiliary moiety; and n = 1 - 4;
b) subjecting said chiral compound of Formula III to selective hydrolysis to obtain (R)-5-(methoxycarbonyl)-2,6-dimethyl-4-(3-nitrophenyl)-1,4-dihydro pyridine-3-carboxylic acid; and
c) converting said (R)-5-(methoxycarbonyl)-2,6-dimethyl-4-(3-nitrophenyl)-1,4-dihydropyridine-3-carboxylic acid to Barnidipine or a pharmaceutically acceptable salt thereof,
wherein said chiral compound of Formula III is selected from (2S,3S)-dimethyl-2,3-bis[(4S)-5-methoxycarbonyl- 2,6-dimethyl- 4-(3-nitrophenyl)- 1,4-dihydropyridine- 3-carboxy]butanedioate, (2S,3S)-diethyl- 2,3-bis[(4S)-5-methoxycarbonyl-2,6-dimethyl-4-(3-nitrophenyl)-1,4-dihydropyridine-3-carboxy] butanedioate, (1R,2S,5R)-2-isopropyl-5-methyl-1-[(4S)-5-methoxy carbonyl-2,6-dimethyl-4-(3-nitrophenyl)- 1,4-dihydropyridine- 3-carboxy]cyclohexane, (S)-alpha[(4S)-5-methoxycarbonyl- 2,6-dimethyl- 4-(3-nitrophenyl)-1,4-dihydropyridine-3-carboxy]phenyl acetic acid methyl ester, (S)-alpha [(4S)-5-methoxycarbonyl-2,6-dimethyl-4-(3-nitrophenyl)-1,4-dihydro pyridine-3-carboxy] phenyl acetic acid ethyl ester, Dimethyl-(S)-alpha [(4S)-5-methoxycarbonyl-2,6-dimethyl- 4-(3-nitrophenyl)- 1,4-dihydropyridine-3-carboxy]butanedioate or Diethyl-(S)-alpha [(4S)-5-methoxycarbonyl- 2,6-dimethyl- 4-(3-nitrophenyl)-1,4-dihydropyridine-3-carboxy]butanedioate.

Preferably, the selective hydrolysis is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxyethane, polyethylene glycol or water.

Another aspect of the present invention provides conversion of (R)-5-(methoxycarbonyl)- 2,6-dimethyl- 4-(3-nitrophenyl)- 1,4-dihydropyridine-3-carboxylic acid to Barnidipine or a pharmaceutically acceptable salt thereof by a process comprising the steps of,
a) coupling (R)-5-(methoxycarbonyl)- 2,6-dimethyl- 4-(3-nitrophenyl)- 1,4-dihydropyridine-3-carboxylic acid with (S)-1-benzyl-3-hydroxypyrrolidine in presence of an acid activating agent selected from thionyl chloride, phosphorus pentachloride, oxalyl chloride, pivaloyl chloride, N,N'-dicyclohexylcarbodiimide (DCC), 1,1'-carbonyldiimidazole(CDI) or 3-(ethyliminomethyleneamino)-N,N-dimethylpropan-1-amine(EDC) to obtain Barnidipine;
b) optionally converting said Barnidipine to a pharmaceutically acceptable salt thereof.

Preferably, Barnidipine is purified by a process comprising the steps of,
a) treating Barnidipine with a salt forming agent to obtain Barnidipine acid addition salt;
b) purifying said Barnidipine acid addition salt; and
c) converting pure Barnidipine acid addition salt to Barnidipine.

Preferably, said salt forming agent is a chiral resolving agent.

Another aspect of the present invention provides a process for preparation of Barnidipine acid addition salt, wherein the process comprises treating Barnidipine with a chiral agent to obtain Barnidipine acid addition salt, wherein said chiral agent is selected from L-malic acid, D-malic acid, L-tartaric acid, D-tartaric acid, Dibenzoyl-L-tartaric acid, Dibenzoyl-D-tartaric acid, Di-p-toluoyl-L-tartaric acid or Di-p-toluoyl-D-tartaric acid.

Another aspect of the present invention provides a process for preparation of (R)-5-(methoxycarbonyl)-2,6-dimethyl-4-(3-nitrophenyl)-1,4-dihydropyridine-3-carboxylic acid, comprising the steps of,
a) providing a chiral compound of Formula II or Formula III, 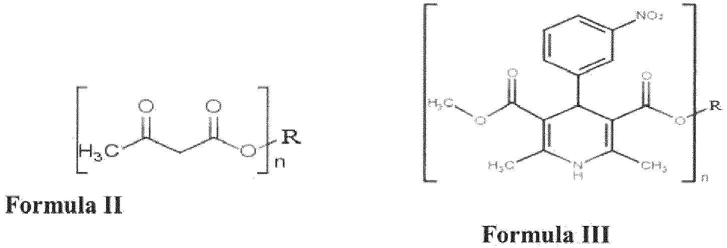 where R is a chiral auxiliary moiety; and n = 1 - 4; and
b) converting said chiral compound of Formula II or Formula III to (R)-5-(methoxycarbonyl)-2,6-dimethyl-4-(3-nitrophenyl)-1,4-dihydropyridine-3-carboxylic acid, wherein said chiral auxiliary moiety is selected from the group consisting of (2S, 3S)-disubstituted butanedioate, (1R,2S, 5R)-2-isopropyl-5-methyl-cyclohexyl, N-benzyl-3(S)-pyrrolidinyl, (S)-alpha-substituted phenyl acetic acid ester and (S)-alpha-substituted butanedioate.

Preferably, said (R)-5-(methoxycarbonyl)-2,6-dimethyl-4-(3-nitrophenyl)-1,4-dihydro pyridine-3-carboxylic acid is converted to Barnidipine or a pharmaceutically acceptable salt thereof. 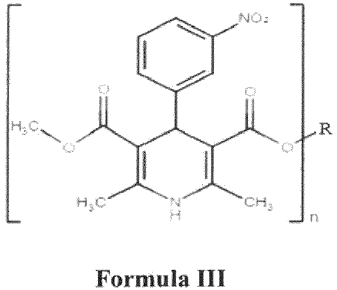 where R is a chiral auxiliary moiety selected from the group consisting of (2S,3S)-disubstituted butanedioate, (1R,2S,5R)-2-isopropyl-5-methyl-cyclohexyl, (S)-alpha-substituted phenyl acetic acid ester and (S)-alpha substituted butanedioate; and n=1 or 2.

Preferably, said compound of Formula III is selected from Formula III A, III B, III D or III E,

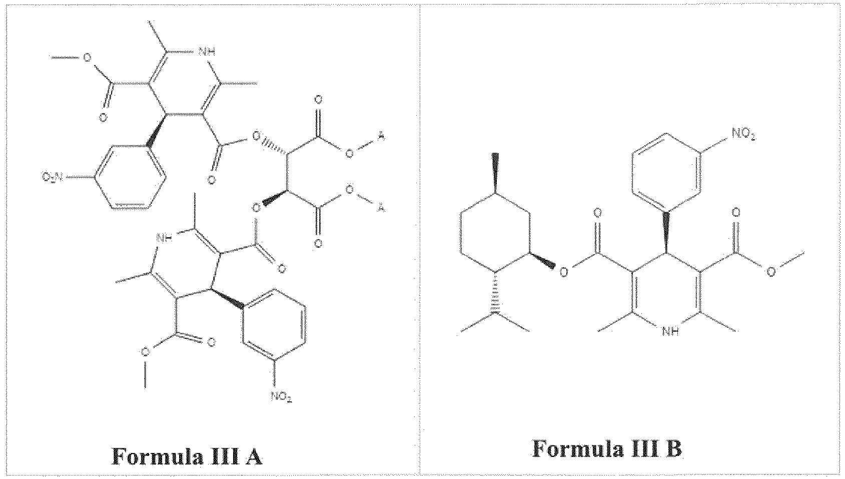

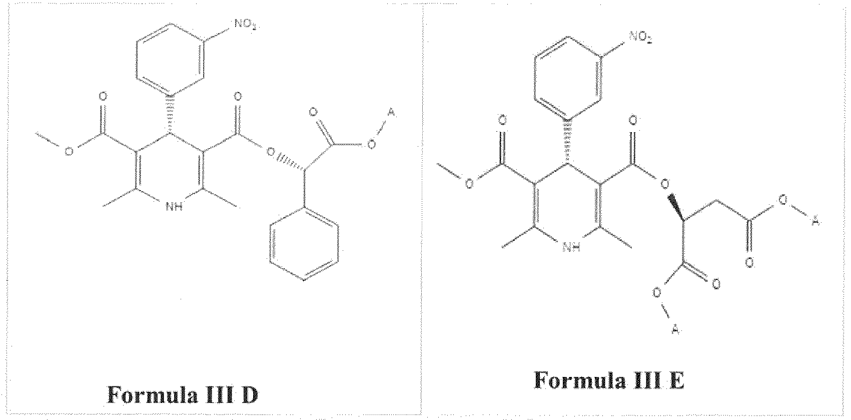

where A is selected from methyl, ethyl, n-propyl, isopropyl or benzyl.

Another aspect of the present invention provides use of a compound of Formula II and/or Formula III in the preparation of Barnidipine or a pharmaceutically acceptable salt thereof.

Preferably for said use the compound of Formula III is subjected to selective hydrolysis in the presence of ethylene glycol so as to prepare Barnidipine or a pharmaceutically acceptable salt thereof.

### Brief Description of the Drawings:

Fig.1: X-ray Diffraction pattern of Barnidipine hydrochloride Form I obtained according to the present invention.
Fig.2: X-ray Diffraction pattern of Barnidipine obtained according to the present invention.

### Detailed Description of the Invention:

The present invention provides a simple, cost effective and industrially viable process for the preparation of Barnidipine or a pharmaceutically acceptable salt thereof, in particular Barnidipine hydrochloride or Barnidipine malate.

According to one embodiment of the present invention, there is provided a process for preparation of Barnidipine or a pharmaceutically acceptable salt thereof, comprising the steps of,
a) providing a chiral compound of Formula II, 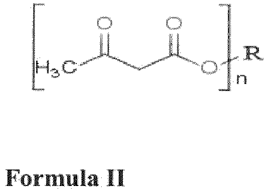 where R is a chiral auxiliary moiety; and n = 1 - 4;
b) treating said chiral compound of Formula II with m-nitrobenzaldehyde and methyl-3-aminocrotonate under Hantzsch reaction conditions to obtain a chiral compound of Formula III; 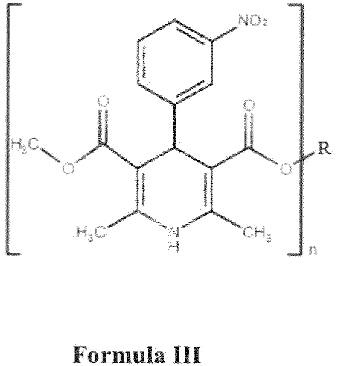 where R is a chiral auxiliary moiety; and n = 1 - 4; and
c) converting said chiral compound of Formula III to Barnidipine or a pharmaceutically acceptable salt thereof, wherein said chiral auxiliary moiety is selected from the group consisting of
   (2S,3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)-alpha substituted butanedioate. Preferably, n = 1 or 2.

The chiral compound of Formula II is prepared by reacting a chiral auxiliary reactant with diketene, acetoacetic acid or a derivative thereof.

A chiral auxiliary reactant is a stereogenic unit which is temporarily incorporated into an organic compound to control the stereochemical outcome of the synthesis. Any suitable chiral auxiliary reactant can be used.

Preferably, the chiral auxiliary reactant is a chiral alcohol or derivative thereof.

In a preferred embodiment, the chiral auxiliary reactant is selected from 2S,3S-dihydroxybutanedioic acid, 2S,3S-dihydroxybutanedioic acid ester, (1R,2S,5R)-2-isopropyl-5-methyl-cyclohexanol, N-benzyl-3(S)-hydroxypyrrolidine, (S)-alpha hydroxy phenyl acetic acid, (S)-alpha hydroxy phenyl acetic acid ester, (S)- alpha hydroxy butanedioic acid or (S)-alpha hydroxy butanedioic acid ester.

Preferably, the acetoacetic acid derivative is selected from acetoacetic acid ester or 2,2,6-Trimethyl-4H-1,3-dioxin-4-one (also referred to as Diketene acetone adduct).

The reaction of a chiral auxiliary reactant with diketene, acetoacetic acid or a derivative thereof may be carried out in the presence of a catalyst such as 4-DMAP or any other suitable tertiary amine.

In a preferred embodiment, the chiral auxiliary reactant is reacted with diketene, acetoacetic acid ester or diketene acetone adduct in an inert solvent selected from toluene, xylene, THF, 1,4-dioxane or DIPE, preferably toluene or xylene, to obtain the corresponding chiral compound of Formula II.

Preferably, a chiral compound of Formula II is selected from (2S,3S)-dimethyl 2,3-bis(3-oxobutanoyloxy)butanedioate, (2S,3S)-diethyl 2,3-bis(3-oxobutanoyloxy) butanedioate, (1R,2S,5R)-2-isopropyl-5-methyl-1-(3-oxobutanoyloxy)cyclohexane, (3S)-(acetoacetoxy)-1-benzylpyrrolidine, (S)-alpha (acetoacetoxy) phenyl acetic acid methyl ester, (S)-alpha (acetoacetoxy) phenyl acetic acid ethyl ester, Dimethyl-(S)-alpha(acetoacetoxy)-butanedioate or Diethyl-(S)-alpha (acetoacetoxy)-butanedioate. More preferably, the chiral compound of Formula II is selected from (2S,3S)-dimethyl 2,3-bis(3-oxobutanoyloxy)butanedioate or (2S,3S)-diethyl 2,3-bis(3-oxobutanoyloxy) butanedioate, also referred to as (2S,3S)-2,3-bis(3-oxobutanoyloxy) succinic acid ester.

In a preferred embodiment, 2,2,6-trimethyl-4H-1,3-dioxin-4-one is added to a solution or suspension of 2S,3S-dihydroxybutanedioic acid ester in toluene to obtain a mixture. The resulting mixture is heated to reflux for about 4-10 hours, preferably 5-8 hours. The reaction mixture is then cooled and the corresponding compound of Formula II, i.e., (2S,3S)-2,3-bis(3-oxobutanoyloxy)butanedioate, thus obtained is isolated. The product obtained is used as such for the further reaction, without any purification.

In a preferred embodiment, a chiral compound of Formula II is subjected to Hantzsch reaction to obtain the corresponding compound of Formula III. Preferably, a chiral compound of Formula II is reacted with m-nitrobenzaldehyde and methyl-3-aminocrotonate in presence of a solvent selected from methanol, ethanol, n-propanol, isopropanol, n-butanol, toluene, acetonitrile, DMF, cyclohexane or ethylene glycol (also referred to as monoethylene glycol) under reflux conditions for about 4 -10 hours, preferably about 5 - 6 hours. The obtained reaction mixture is cooled and maintained under stirring for about 0.5 - 2 hours, preferably one hour. The reaction mixture is filtered and the product thus obtained is isolated. Compound of Formula III, thus obtained, is used as such for the further reaction, without any purification.

Preferably, the Hantzsch reaction is carried out in the presence of ammonium acetate.

Another embodiment of the present invention provides a process for conversion of compound of Formula III to Barnidipine or a pharmaceutically acceptable salt thereof, the process comprising the steps of,
a) subjecting a chiral compound of Formula III, 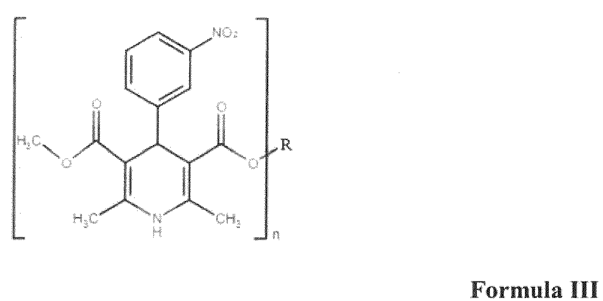 where R is a chiral auxiliary moiety; and n = 1 - 4;
   to selective hydrolysis to obtain (R)-5-(methoxycarbonyl)-2,6-dimethyl-4-(3-nitro plienyl)-1,4-dihydropyridine-3-carboxylic acid; and
b) converting said (R)-5-(methoxycarbonyl)-2,6-dimethyl-4-(3-nitrophenyl)-1,4-dihydropyridine-3-carboxylic acid to Barnidipine or a pharmaceutically acceptable salt thereof, wherein said chiral auxiliary moiety is selected from the group consisting of (2S,3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)-alpha substituted butanedioate. Preferably, n = 1 or 2.

Preferably, said compound of Formula III is selected from the group consisting of (2S,3S)-dimethyl-2,3-bis[(4S)-5-methoxycarbonyl-2,6-dimethyl-4-(3-nitrophenyl)-1,4-dihydropyridine-3-carboxy]butanedioate, (2S,3S)-diethyl-2,3-bis[(4S)-5-methoxy carbonyl-2,6-dimethyl- 4-(3-nitrophenyl)-1,4-dihydropyridine-3-carboxy] butanedioate, (1R,2S,5R)-2-isopropyl-5-methyl-1-[(4S)-5-methoxycarbonyl-2,6-dimethyl-4-(3-nitrophenyl)-1,4-dihydropyridine-3-carboxy]cyclohexane, (S)-alpha [(4S)-5-methoxycarbony]-2,6-dimethyl -4-{3-nitrophenyl)-1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydro pyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy] butanedioate and Diethyl-(S)-alpha [(4S)-5-methoxycarbonyl-2,6-dimethyl-4-(3-nitrophenyl)-1,4-dihydropyridine-3-carboxy] butanedioate.

Preferably, the selective hydrolysis is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxy ethane, polyethylene glycol or water to yield the desired isomer.

According to one embodiment, the process for preparation of Barnidipine or a pharmaceutically acceptable salt thereof is as represented in Scheme 1 below, 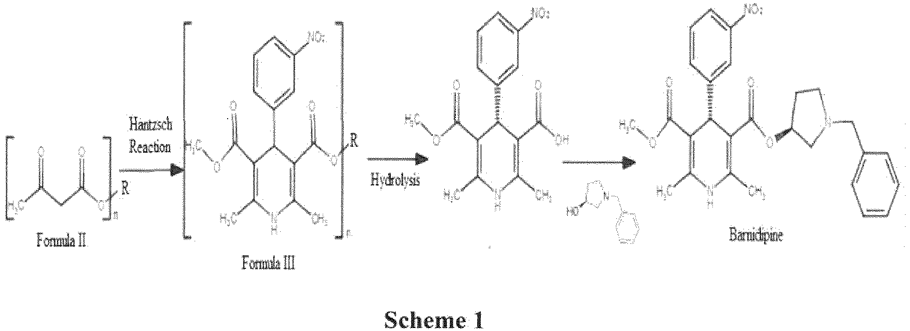 (R)-5-(methoxycarbonyl)-2,6-dimethyl-4-(3-nitrophenyl)-1,4-dihydropyridine-3-carboxylic acid is converted to Barnidipine or a pharmaceutically acceptable salt thereof, by processes known in the art or by the process described herein.

The present specification also describes a chiral compound of Formula II, 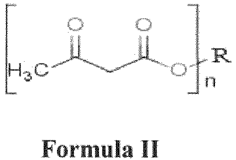 where R is a chiral auxiliary moiety as defined above; and n = 1 - 4.

Preferably, R is a chiral auxiliary moiety selected from the group consisting of (2S,3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)-alpha substituted butanedioate. Preferably, n = 1 or 2.

Preferably, said chiral compound of Formula II is selected from Formula IIA, IIB, IIC, IID or IIE.

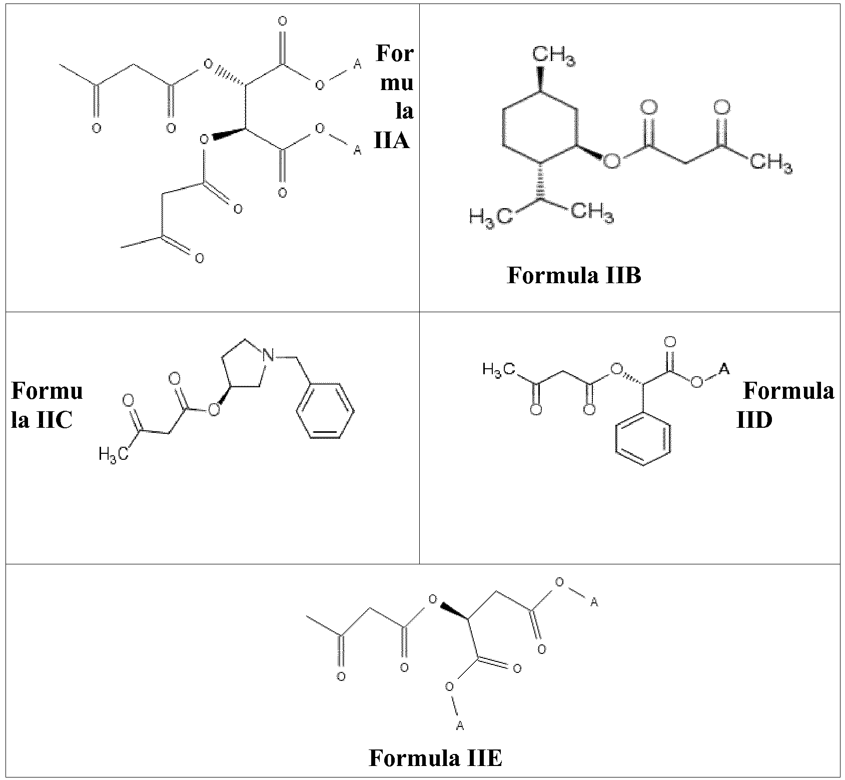

where A is selected from C₁-C₅ alkyl, aryl or aralkyl.

Preferably, A is selected from methyl, ethyl, n-propyl, isopropyl or benzyl.

Mixed esters of compound of Formula IIA and IIE are also included within the scope of this invention.

In a preferred embodiment, a chiral compound of Formula II is selected from (2S,3S)-dimethyl 2,3-bis(3-oxobutanoyloxy)butanedioate, (2S,3S)-diethyl 2,3-bis(3-oxobutanoyloxy)butanedioate, (1R,2S,5R)-2-isopropyl-5-methyl-1-(3-oxo butanoyl oxy)cyclohexane, (3S)-(acetoacetoxy)-1-benzylpyrrolidine, (S)-alpha (acetoacetoxy) phenyl acetic acid methyl ester, (S)-alpha (acetoacetoxy) phenyl acetic acid ethyl ester, Dimethyl-(S)-alpha(acetoacetoxy)-butanedioate or Diethyl-(S)-alpha (acetoacetoxy)-butanedioate.

Another embodiment of the present invention provides a chiral compound of Formula III, 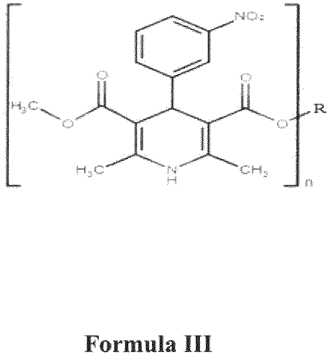 where R is a chiral auxiliary moiety; and n = 1 - 4, wherein said chiral auxiliary moiety is selected from the group consisting of (2S,3S)-disubstituted butanedioate, (1R,2S,5R)-2-isopropyl-5-methyl-cyclohexyl, (S)-alpha-substituted phenyl acetic acid ester and (S)-alpha substituted butanedioate. Preferably, n = 1 or 2.

In a preferred embodiment, the chiral compound of Formula III is selected from Formula IIIA, III B, III D or III E.

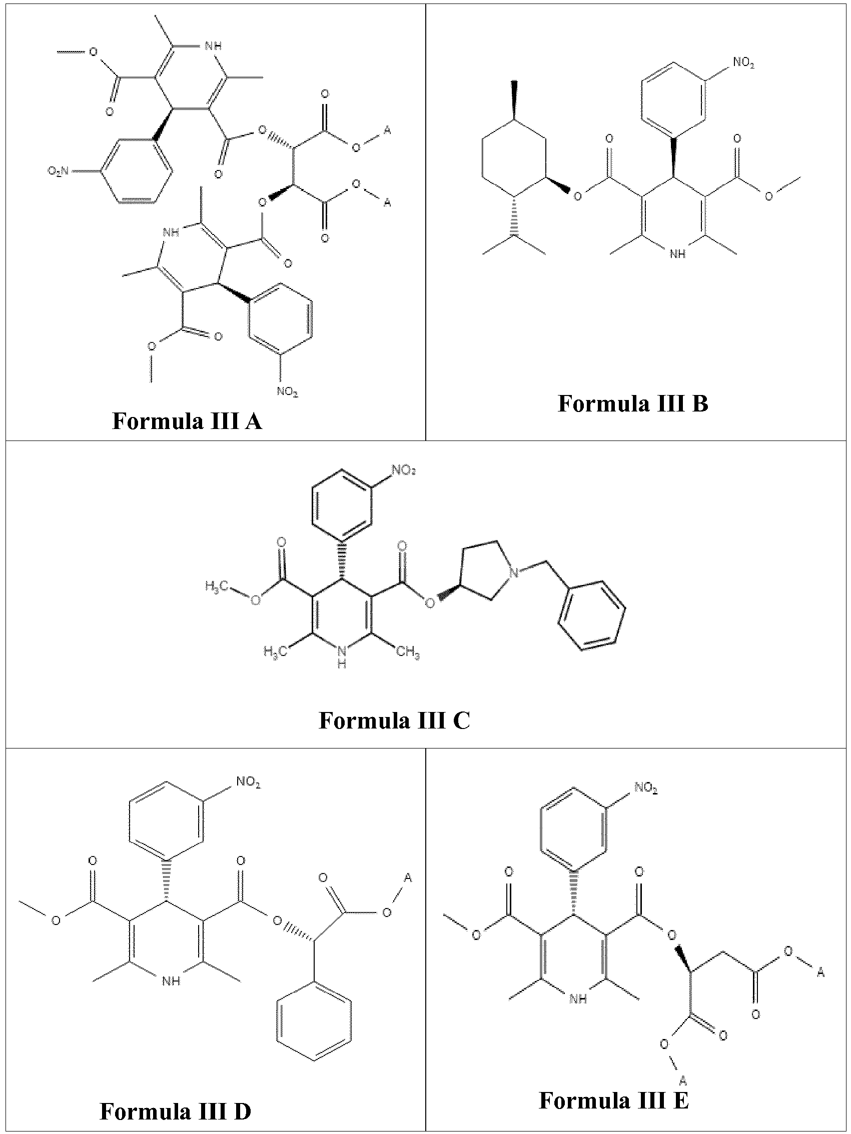

where A is selected from C₁-C₅ alkyl, aryl or aralkyl.

Preferably, A is selected from methyl, ethyl, n-propyl, isopropyl or benzyl.

Mixed esters of compound of Formula IIIA and IIIE are also included within the scope of this invention.

In a preferred embodiment, the chiral compound of Formula III is selected from the group consisting of, (2S,3S)-dimethyl-2,3-bis[(4S)-5-methoxycarbonyl-2,6-dimethyl-4-(3-nitrophenyl)-1,4-dihydropyridine-3-carboxy] butanedioate, (2S,3S)-diethyl-2,3-bis[(4S)-5-methoxycarbonyl-2,6-dimethyl-4-(3-nitrophenyl)-1,4-dihydropyridine-3-carboxy] butanedioate, (1R,2S,5R)-2-isopropyl-5-methyl-1-[(4S)-5-methoxycarbonyl-2,6-dimethyl-4-(3-nitrophenyl)-1,4-dihydropyridine-3-carboxy]cyclohexane, (3'S,4S)-1-benzyl-3-pyrrolidinyl-methyl-1,4-dihydro-2,6-dimethyl-4-(3-nitrophenyl)-3 ,5-pyridine dicarboxylate, (S)-alpha [(4S)-5-methoxycarbonyl-2,6-dimethyl-4-(3-nitrophenyl)-1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl- 4-(3-nitrophenyl)- 1,4-dihydropyridine-3-carboxy] butanedioate and Diethyl-(S)-alpha [(4S)-5-methoxycarbonyl-2,6-dimethyl-4-(3-nitrophenyl)-1,4-dihydropyridine-3-carboxy] butanedioate.

More preferably, compound of Formula III is (2S,3S)-dimethyl-2,3-bis[(4S)-5-methoxycarbonyl-2,6-dimethyl-4-(3-nitrophenyl)-1,4-dihydropyridine-3-carboxy] butanedioate or (2S,3S)-diethyl-2,3-bis[(4S)-5-methoxycarbonyl-2,6-dimethyl-4-(3-nitrophenyl)-1,4-dihydropyridine-3-carboxy] butanedioate.

Other (2S,3S)-dialkyl or diaryl-2,3-bis[(4S)-5-methoxycarbonyl-2,6-dimethyl-4-(3-nitrophenyl)-1,4-dihydropyridine-3-carboxy] butanedioate are also covered within the scope of this invention.

In a preferred embodiment of the present invention, the process for preparation of Barnidipine or a pharmaceutically acceptable salt thereof, comprises the steps of:
a) treating (2S,3S)-dimethyl-2,3-bis(3-oxobutanoyloxy)butanedioate with m-nitrobenzaldehyde and methyl-3-aminocrotonate under Hantzsch reaction conditions to obtain (2S,3S)-dimethyl-2,3-bis[(4S)-5-methoxycarbonyl-2,6-dimethyl-4-(3-nitrophenyl)-1,4-dihydropyridine-3-carboxy] butanedioate;
b) subjecting said (2S,3S)-dimethyl-2,3-bis[(4S)-5-methoxycarbonyl-2,6-dimethyl-4-(3-nitrophenyl)-1,4-dihydropyridine-3-carboxy] butanedioate to selective hydrolysis to obtain (R)-5-(methoxycarbonyl)-2,6-dimethyl-4-(3-nitrophenyl)-1,4-dihydropyridine-3-carboxylic acid; and
c) converting said (R)-5-(methoxycarbonyl)-2,6-dimethyl-4-(3-nitrophenyl)-1,4-dihydropyridine-3-carboxylic acid to Barnidipine or a pharmaceutically acceptable salt thereof.

In a preferred embodiment, the process for the preparation of Barnidipine or a pharmaceutically acceptable salt thereof, comprises the steps of,

Step I involves treating (2S,3S)-dimethyl-2,3-bis(3-oxobutanoyloxy) butanedioate with m-nitrobenzaldehyde and methyl-3-aminocrotonate under Hantzsch reaction conditions to obtain (2S,3S)-dimethyl-2,3-bis[(4S)-5-methoxycarbonyl-2,6-dimethyl-4-(3-nitrophenyl)-1,4-dihydropyridine-3-carboxy] butanedioate.

A mixture of (2S,3S)-dimethyl-2,3-bis(3-oxobutanoyloxy)butanedioate, m-nitrobenzaldehyde, methyl-3-aminocrotonate and ammonium acetate in a solvent selected from methanol, ethanol, n-propanol, isopropanol, n-butanol, toluene, acetonitrile, DMF, cyclohexane or monoethylene glycol, preferably methanol is heated to reflux for about 4-10 hours, preferably about 4-8 hours, more preferably for about 5-6 hours. The obtained reaction mixture is cooled to 0 to 20°C, preferably 10 to 15°C and maintained under stirring at the same temperature for about 0.5 to 2 hours, preferably for 1 hour. The reaction mixture is filtered to obtain a wet cake. The obtained wet cake is treated with a solvent selected from methanol, ethanol, n-propanol, isopropanol or n-butanol, preferably methanol to obtain a slurry. The slurry is filtered and product obtained is dried.

(2S,3S)-dimethyl-2,3-bis[(4S)-5-methoxycarbonyl-2,6-dimethyl-4-(3-nitrophenyl)-1,4-dihydropyridine-3-carboxy] butanedioate [also known as (4S,4'S)-O'³,O³-((2S,3S)-1,4-dimethoxy-1,4-dioxobutane-2,3-diyl)-5-dimethyl bis(2,6-dimethyl-4-(3-nitrophenyl)-1,4-dihydropyridine-3,5-dicarboxylate or (2S,3S)-dimethyl-2,3-bis[(4S)-5-methoxycarbonyl-2,6-dimethyl-4-(3-nitrophenyl)-1,4-dihydropyridine-3-carboxy] succinate] obtained by the process of the present invention exhibits the desired chemical purity and/or chiral purity.

Step II involves hydrolyzing (2S,3S)-dimethyl-2,3-bis[(4S)-5-methoxy carbonyl-2,6-dimethyl-4-(3-nitrophenyl)-1,4-dihydropyridine-3-carboxy] butanedioate to obtain (R)-5-(methoxycarbonyl)-2,6-dimethyl- 4-(3-nitrophenyl)- 1,4-dihydropyridine-3-carboxylic acid.

(2S,3S)-dimethyl- 2,3-bis[(4S)-5-methoxycarbonyl- 2,6-dimethyl-4-(3-nitrophenyl) - 1,4-dihydropyridine-3-carboxy] butanedioate is treated with a base selected from potassium hydroxide, sodium hydroxide, lithium hydroxide, calcium hydroxide or cesium hydroxide, preferably potassium hydroxide and solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxyethane, polyethylene glycol or water, preferably, ethylene glycol to obtain a reaction mixture. The obtained reaction mixture is heated to about 50 to 70°C, preferably about 60 to 70°C, more preferably about 65 to 70°C for about 2 to 5 hours, preferably for about 3 hours. The reaction mixture is cooled to 20 to 40°C, preferably 25 to 30°C and then diluted with water and extracted with a solvent selected from dichloromethane, dichloroethane, chloroform or carbon tetrachloride, preferably dichloromethane. The layers are separated. The pH of the aqueous layer is adjusted to 4 to 5 by addition of a suitable acid. The solid obtained is filtered, washed with water and dried to obtain (R)-5-(methoxycarbonyl)-2,6-dimethyl-4-(3-nitrophenyl)-1,4-dihydropyridine-3-carboxylic acid.

Step III involves conversion of (R)-5-(methoxycarbonyl)-2,6-dimethyl-4-(3-nitrophenyl)-1,4-dihydropyridine-3-carboxylic acid to Barnidipine or a pharmaceutically acceptable salt thereof, comprising the steps of:
a) coupling (R)-5-(methoxycarbonyl)- 2,6-dimethyl- 4-(3-nitrophenyl)-1,4-dihydropyridine-3-carboxylic acid with (S)-1-benzyl-3-hydroxypyrrolidine in presence of an acid activating agent to obtain Barnidipine; and
b) converting Barnidipine to a pharmaceutically acceptable salt thereof.

Acid activating agent is selected from thionyl chloride, phosphorus pentachloride, oxalyl chloride, pivaloyl chloride, N,N'-dicyclohexylcarbodiimide (DCC), 1,1'-carbonyldiimidazole (CDI), 3-(ethyliminomethyleneamino)-N,N-dimethylpropan-1-amine (EDC) or the like.

In one embodiment, (R)-5-(methoxycarbonyl)-2,6-dimethyl-4-(3-nitrophenyl)-1,4-dihydropyridine-3-carboxylic acid is treated with a solvent selected from dimethyl formamide, dimethylacetamide, dichloromethane, tetrahydrofuran, dimethyl sulfoxide or a mixture thereof to obtain a suspension. Acid activating agent such as thionyl chloride or phosphorus pentachloride, preferably thionyl chloride is added to the suspension at -15 to 15°C, preferably at -10 to 0°C. The obtained reaction mixture is maintained under stirring for 1 to 3 hours, preferably for 2 hours. A solution of (S)-1-benzyl-3-hydroxypyrrolidine in a solvent selected from dichloromethane, dichloroethane, chloroform or carbon tetrachloride, preferably dichloromethane is added dropwise to the reaction mixture at -20 to -10°C. The mixture is maintained under stirring for 2 to 6 hours, preferably for 3 hours. The mixture is quenched in water and then subjected to layer separation. The organic layer is washed with aqueous bicarbonate solution, water, dried over sodium sulphate and then concentrated to obtain (3'S,4S)-1-benzyl-3-pyrrolidinyl-methyl-1,4-dihydro-2,6-dimethyl-4-(3-nitrophenyl)-3,5-pyridine dicarboxylate as an oil.

In another embodiment, a mixture of (R)-5-(methoxycarbonyl)-2,6-dimethyl-4-(3-nitrophenyl)- 1,4-dihydropyridine- 3-carboxylic acid, (S)-1-benzyl-3-hydroxy pyrrolidine, acid activating agent such as N,N'-dicyclohexylcarbodiimide (DCC) and a catalytic amount of 4-dimethylaminopyridine in a solvent selected from toluene, xylene, pentane, hexane or heptane, preferably toluene is refluxed for 2 to 4 hours, preferably for 3 hours. The obtained reaction mixture is maintained under stirring for about 0.5 to 5 hours, preferably for 1 to 3 hours at room temperature. The reaction mixture is filtered and the filtrate is washed with aqueous bicarbonate solution, water, dried over sodium sulphate and then concentrated to obtain (3'S,4S)-1-benzyl-3-pyrrolidinyl-methyl- 1,4-dihydro- 2,6-dimethyl- 4-(3-nitrophenyl) -3,5-pyridine dicarboxylate as an oil.

(3'S,4S)-1-benzyl-3-pyrrolidinyl-methyl-1,4-dihydro-2,6-dimethyl-4-(3-nitrophenyl) -3,5-pyridine dicarboxylate is then converted to a pharmaceutically acceptable salt, in particular Barnidipine hydrochloride or Barnidipine malate.

Another embodiment of the present invention provides a process for preparation of Barnidipine or a pharmaceutically acceptable salt thereof comprising the steps of,
a) providing a chiral compound of Formula III, 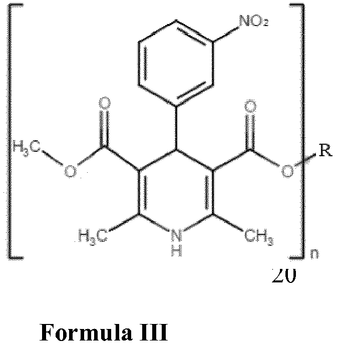 where R is a chiral auxiliary moiety; and n = 1 - 4;
b) subjecting said chiral compound of Formula III to selective hydrolysis to obtain (R)-5-(methoxycarbonyl)-2,6-dimethyl- 4-(3-nitrophenyl)- 1,4-dihydropyridine-3-carboxylic acid; and
c) converting said (R)-5-(methoxycarbonyl)-2,6-dimethyl-4-(3-nitrophenyl)-1,4-dihydropyridine-3-carboxylic acid to Barnidipine or a pharmaceutically acceptable salt thereof,
wherein said chiral compound of Formula III is selected from (2S,3S)-dimethyl-2,3-bis[(4S)-5-methoxycarbonyl-2,6-dimethyl-4-(3-nitrophenyl)-1,4-dihydropyridine-3-carboxy] butanedioate, (2S,3S)-diethyl-2,3-bis[(4S)-5-methoxycarbonyl-2,6-dimethyl-4-(3-nitrophenyl)-1,4-dihydropyridine-3-carboxy]butanedioate, (1R,2S,5R)-2-isopropyl-5-methyl-1-[(4S)-5-methoxycarbonyl-2,6-dimethyl-4-(3-nitrophenyl)-1,4-dihydropyridine-3-carboxy] cyclohexane, (S)-alpha [(4S)-5-methoxycarbonyl-2,6-dimethyl-4-(3-nitrophenyl)-1,4-dihydropyridine-3-carboxy]phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy] butanedioate or Diethyl-(S)-alpha [(4S)-5-methoxycarbonyl-2,6-dimethyl-4-(3-nitrophenyl)-1,4-dihydropyridine-3-carboxy]butanedioate.

Preferably, the selective hydrolysis is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxy ethane, polyethylene glycol or water to yield the desired isomer.

In a preferred embodiment, the selective hydrolysis is carried out in the presence of ethylene glycol at a temperature of about 50 to 70°C.

The use of diols, in particular, ethylene glycol affords the selective hydrolysis of the desired ester bond thereby controlling the generation of hydrolysis impurities. Further generation of impurities by transesterification is controlled by adjusting the volume of ethylene glycol with respect to the chiral compound of Formula III. Ethylene glycol is used in an amount of two to four volumes, preferably three volumes with respect to the chiral compound of Formula III.

Preferably, compound of Formula III is (2S,3S)-dimethyl-2,3-bis[(4S)-5-methoxycarbonyl- 2,6-dimethyl- 4-(3-nitrophenyl)- 1,4-dihydropyridine-3-carboxy] butanedioate or (2S,3S)-diethyl-2,3-bis[(4S)-5-methoxycarbonyl- 2,6-dimethyl- 4-(3-nitrophenyl)- 1,4-dihydropyridine-3-carboxy]butanedioate

According to one embodiment of the present invention, the process for preparation of Barnidipine or a pharmaceutically acceptable salt thereof, is as represented in Scheme 2 below,

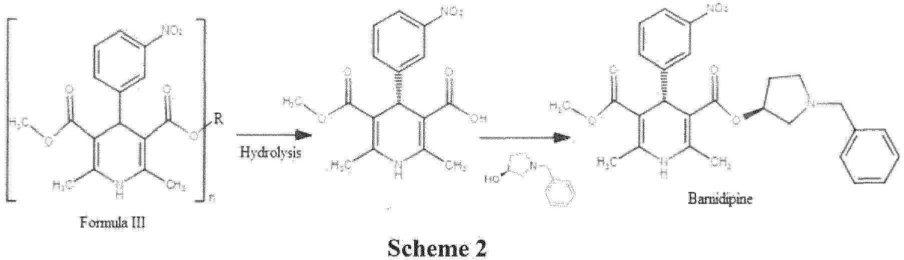

Yet another embodiment of the present invention provides the use of ethylene glycol in the preparation of Barnidipine or a pharmaceutically acceptable salt thereof.

Another embodiment of the present invention provides a process for preparation of (R)-5-(methoxycarbonyl)-2,6-dimethyl-4-(3-nitrophenyl)-1,4-dihydropyridine-3-carboxylic acid comprising the steps of,
a) providing a chiral compound of Formula
   II or Formula III 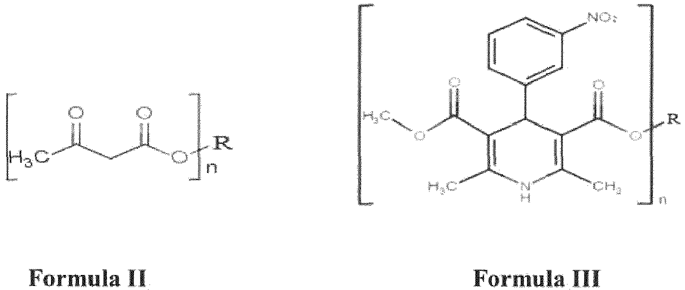 where R is a chiral auxiliary moiety; and n = 1 - 4; and
b) converting said chiral compound of Formula II or Formula III to (R)-5-(methoxycarbonyl)- 2,6-dimethyl- 4-(3-nitrophenyl)- 1,4-dihydropyridine-3-carboxylic acid, wherein said chiral auxiliary moiety is selected from the group consisting of (2S,3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)-alpha substituted butanedioate. Preferably, n = 1 or 2.

The process disclosed in the prior art involves resolution of (±)-5-(methoxy carbonyl)-2,6-dimethyl-4-(3-nitrophenyl)-1,4-dihydropyridine-3-carboxylic acid to obtain the desired R-isomer. Further the prior art process involves the use of column chromatography. The process of the present invention involves the principle of asymmetric induction and/or diastereomeric crystallization.

Asymmetric induction, also known as enantioinduction, in stereochemistry describes the preferential formation in a chemical reaction of one enantiomer or diastereoisomer over the other as a result of the influence of a chiral feature present in the substrate, reagent, catalyst or environment.

The present invention provides a cost-effective process for the large scale preparation of stereochemically pure (R)-5-(methoxycarbonyl)-2,6-dimethyl-4-(3-nitrophenyl)-1,4-dihydropyridine-3-carboxylic acid, a key intermediate in the synthesis of Barnidipine. (R)-5-(methoxycarbonyl)- 2,6-dimethyl- 4-(3-nitrophenyl)-1,4-dihydropyridine-3-carboxylic acid obtained by the process of the present invention has chemical purity of more than 96% and chiral purity of more than 99%. In particular, the process for the preparation of (R)-5-(methoxycarbonyl)-2,6-dimethyl-4-(3-nitrophenyl)-1,4-dihydropyridine-3-carboxylic acid, as described herein, does not involve any resolution or purification, thereby making the process for preparation of Barnidipine cost-effective and industrially feasible.

The present invention involves the use of a chiral compound of Formula II as a starting material for the preparation of Barnidipine or a pharmaceutically acceptable salt thereof. The use of a chiral starting material improves the yield and purity of the final product.

In a further embodiment, (R)-5-(methoxycarbonyl)-2,6-dimethyl-4-(3-nitrophenyl)-1,4-dihydropyridine-3-carboxylic acid is converted to Barnidipine or a pharmaceutically acceptable salt thereof by processes known in the art or by the process described herein.

Another embodiment of the present invention provides a process for preparation of Barnidipine or a pharmaceutically acceptable salt thereof comprising the steps of,
a) providing a chiral compound of Formula II, 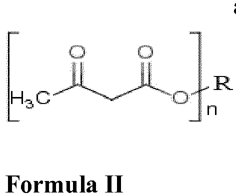 where R is a chiral auxiliary moiety; and n = 1 - 4;
b) treating said chiral compound of Formula II with m-nitrobenzaldehyde and methyl-3-aminocrotonate under Hantzsch reaction conditions to obtain a chiral compound of Formula III; 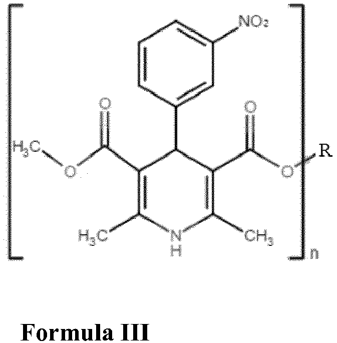 where R is a chiral auxiliary moiety; and n = 1 - 4; and
c) isolating said chiral compound of Formula III,
wherein said compound of Formula II is (3S)-(acetoacetoxy)-1-benzylpyrrolidine and said compound of Formula III is (3'S,4S)-1-benzyl-3-pyrrolidinyl-methyl-1,4-dihydro-2,6-dimethyl-4-(3-nitrophenyl)-3,5-pyridine dicarboxylate (Barnidipine).

Another embodiment of the present invention provides the use of a chiral compound of Formula II in the preparation of Barnidipine or a pharmaceutically acceptable salt thereof.

Another embodiment of the present invention provides the use of a chiral compound of Formula III in the preparation of Barnidipine or a pharmaceutically acceptable salt thereof.

Yet another embodiment of the present invention provides the use of (S)- 1-benzyl-3-hydroxypyrrolidine as a chiral auxiliary reactant in the preparation of Barnidipine or a pharmaceutically acceptable salt thereof.

Another embodiment of the present invention provides a process for preparation of a chiral compound of Formula III, comprising the steps of,
a) providing a chiral compound of Formula II, 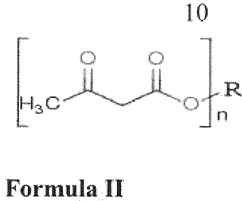 where R is a chiral auxiliary moiety; and n = 1 - 4;
b) treating said chiral compound of Formula II with m-nitrobenzaldehyde and methyl-3-aminocrotonate under Hantzsch reaction conditions to obtain a chiral compound of Formula III; 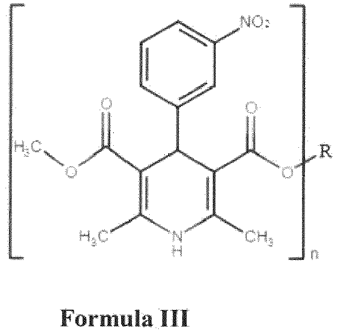 where R is a chiral auxiliary moiety; and n = I - 4; and
c) optionally, isolating said chiral compound of Formula III, wherein said chiral auxiliary moiety is selected from the group consisting of (2S, 3S)-disubstituted butanedioate, (1R,2S, 5R)-2-isopropyt-5-methyl-cyctohexyl, N-benzyl-3(S)-pyrrolidinyl, (S)-alpha-substituted phenyl acetic acid ester and (S)-alpha-substituted butanedioate.

In a preferred embodiment, the chiral compound of Formula III is selected from the group consisting of, (2S,3S)-dimethyl-2,3-bis[(4S)-5-methoxycarbonyl-2,6-dimethyl-4-(3-nitrophenyl)-1,4-dihydropyridine-3-carboxy] butanedioate, (2S,3S)-diethyl-2,3-bis[(4S)-5-methoxycarbonyl-2,6-dimethyl-4-(3-nitrophenyl)-1,4-dihydropyridine-3-carboxy] butanedioate, (1R,2S,5R)-2-isopropyl-5-methyl-1-[(4S)-5-methoxycarbonyl-2,6-dimethyl-4-(3-nitrophenyl)-1,4-dihydropyridine-3-carboxy]cyclohexane, (3'S,4S)-1-benzyl-3-pyrrolidinyl-methyl-1,4-dihydro-2,6-dimethyl-4-(3-nitrophenyl)-3,5-pyridine dicarboxylate, (S)-alpha [(4S)-5-methoxycarbonyl-2,6-dimethyl-4-(3-nitrophenyl)-1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy] butanedioate and Diethyl-(S)-alpha [(4S)-5-methoxycarbonyl-2,6-dimethyl-4-(3-nitrophenyl)-1,4-dihydropyridine-3-carboxy] butanedioate.

More preferably, the chiral compound of Formula III is (2S,3S)-dimethyl-2,3-bis[(4S)-5-methoxycarbonyl-2,6-dimethyl-4-(3-nitrophenyl)-1,4-dihydropyridine-3-carboxy]butanedioate or (2S,3S)-diethyl-2,3-bis[(4S)-5-methoxycarbonyl-2,6-dimethyl-4-(3-nitrophenyl)-1,4-dihydropyridine-3-carboxy] butanedioate.

In a preferred embodiment, the process for preparation of (2S,3S)-dimethyl-2,3-bis[(4S)-5-methoxycarbonyl-2,6-dimethyl-4-(3-nitrophenyl)-1,4-dihydropyridine-3-carboxy] butanedioate, comprises the steps of,
a) treating (2S,3S)-dimethyl-2,3-bis(3-oxobutanoyloxy)butanedioate with m-nitrobenzaldehyde and methyl-3-aminocrotonate under Hantzsch reaction conditions to obtain (2S,3S)-dimethyl-2,3-bis[(4S)-5-methoxycarbonyl-2,6-dimethyl-4-(3-nitrophenyl)-1,4-dihydropyridine-3-carboxy] butanedioate;
b) optionally purifying (2S,3S)-dimethyl-2,3-bis[(4S)-5-methoxycarbonyl-2,6-dimethyl-4-(3-nitrophenyl)-1,4-dihydropyridine-3-carboxy] butanedioate.

Other compounds represented by Formula III can be prepared in a similar manner.

Another embodiment of the present invention provides purification of (3'S,4S)-1-benzyl-3-pyrrolidinyl- methyl- 1,4-dihydro-2,6-dimethyl- 4-(3-nitrophenyl)-3,5-pyridine dicarboxylate (Barnidipine), comprising the steps of,
a) treating Barnidipine with a salt forming agent to obtain Barnidipine acid addition salt;
b) purifying Barnidipine acid addition salt to obtain pure Barnidipine acid addition salt; and
c) converting pure Barnidipine acid addition salt to pure Barnidipine.

In a preferred embodiment, the purification of (3'S,4S)-1-benzyl-3-pyrrolidinylmethyl- 1,4-dihydro-2,6-dimethyl-4-(3-nitrophenyl)-3,5-pyridine dicarboxylate (Barnidipine), comprises the steps of,
a) treating Barnidipine with a suitable solvent to obtain a solution;
b) treating the solution obtained in step a) with a salt forming agent to obtain Barnidipine acid addition salt;
c) purifying Barnidipine acid addition salt to obtain pure Barnidipine acid addition salt; and
d) converting pure Barnidipine acid addition salt to pure Barnidipine.

Solvent is selected from acetone, methyl ethyl ketone, methyl isobutyl ketone, ethanol, methanol, n-propanol, isopropanol, n-butanol, ethyl acetate, methyl acetate, preferably, acetone. Salt forming agent is selected from malic acid, malonic acid, oxalic acid, tartaric acid, formic acid, acetic acid, succinic acid, maleic acid, citric acid, ascorbic acid, mandelic acid, dibenzoyl tartaric acid, methane sulfonic acid, p-toluenesulfonic acid or benzenesulfonic acid, preferably malic acid. Preferably the salt forming agent is a chiral resolving agent.

Another embodiment of the present invention provides a process for preparation of Barnidipine acid addition salt comprising the steps of,
a) treating Barnidipine with a salt forming agent to obtain Barnidipine acid addition salt; and
b) optionally, purifying Barnidipine acid addition salt to obtain pure Barnidipine acid addition salt.

Preferably, the salt forming agent is a chiral agent selected from L-malic acid, D-malic acid, L-tartaric acid, D-tartaric acid, Dibenzoyl-L-tartaric acid, Dibenzoyl-D-tartaric acid, Di-p-toluoyl-L-tartaric acid or Di-p-toluoyl-D-tartaric acid.

In a preferred embodiment, Barnidipine is dissolved in acetone to obtain a solution. To the obtained solution is added, L-malic acid to obtain a mixture. The mixture is maintained under stirring for 2 to 10 hours, preferably for 4 to 8 hours, more preferably 4 hours at room temperature. The mixture is cooled and filtered to obtain Barnidipine malate.

Preferably, Barnidipine malate is dissolved in a solvent selected from methanol, ethanol, n-propanol or isopropanol, preferably methanol to obtain a suspension. The suspension is heated at 60 to 80°C, preferably at 60 to 70°C, more preferably 60 to 65°C to get a clear solution. The solution is concentrated at atmospheric pressure to reduce the volume and maintained under stirring at room temperature for 5 to 10 hours, preferably for 7 to 8 hours. The precipitate thus obtained is filtered, washed and dried to obtain Barnidipine malate having purity greater than 99%.

Barnidipine malate thus obtained is further treated with a solvent selected from methanol, ethanol, n-propanol or isopropanol, preferably methanol to obtain a suspension. The suspension is heated at 60 to 80°C, preferably at 60 to 70°C, more preferably 60 to 65°C to get a clear solution. The solution is concentrated at atmospheric pressure to reduce the volume and further maintained under stirring at room temperature for 5 to 10 hours, preferably for 7 to 8 hours. The precipitate thus obtained is filtered, washed and dried to obtain Barnidipine malate having purity greater than 99.2%.

Barnidipine malate thus obtained is further treated with a solvent selected from methanol, ethanol, n-propanol or isopropanol, preferably methanol to obtain a suspension. The suspension is heated at 60 to 80°C, preferably at 60 to 70°C, more preferably 60 to 65°C to get a clear solution. The solution is treated with activated charcoal and the mixture thus obtained is maintained under stirring at the same temperature for ½ to 1 hour. The mixture is then filtered and the filtrate is concentrated at atmospheric pressure to reduce the volume and further maintained under stirring at room temperature for 5 to 10 hours, preferably for 7 to 8 hours. The precipitate thus obtained is filtered, washed and dried to obtain Barnidipine malate having purity greater than 99.4%.

The above process for purification of Barnidipine malate may be carried out once or may be repeated to obtain Barnidipine malate with the desired purity. Barnidipine malate obtained by the process of the present invention is converted to Barnidipine hydrochloride.

In an alternate embodiment, Barnidipine malate is suspended in about 8 to 10 volumes of solvent to obtain a suspension. The suspension is heated to obtain a hot slurry of Barnidipine malate. This slurry is maintained under stirring at the same temperature and further at room temperature. The slurry is then filtered to obtain Barnidipine malate having the desired purity. Solvent is selected from methanol, ethanol, n-propanol or isopropanol, preferably methanol.

Another embodiment of the present invention provides a process for conversion of Barnidipine malate to Barnidipine hydrochloride, comprising the steps of,
a) treating Barnidipine malate with hydrochloric acid to obtain Barnidipine hydrochloride, and
b) isolating Barnidipine hydrochloride.

Another embodiment of the present invention provides a process for conversion of Barnidipine malate to Barnidipine hydrochloride, comprising the steps of,
a) treating Barnidipine malate with a solvent to obtain a suspension;
b) treating the suspension obtained in step a) with a base to obtain Barnidipine;
c) optionally isolating Barnidipine;
d) treating Barnidipine obtained in step b) or c) with hydrochloric acid to obtain Barnidipine hydrochloride; and
e) isolating Barnidipine hydrochloride.

Preferably, Barnidipine malate is suspended in a solvent selected from dichloromethane, dichloroethane, chloroform or carbon tetrachloride, preferably dichloromethane to obtain a mixture. To this mixture, is added sodium bicarbonate solution and the mixture thus obtained is maintained under stirring at a temperature of 20 to 40°C, preferably at 25 to 30°C for about ½ to 1 hour. The layers are separated. The organic layer is washed with water and then concentrated to obtain an oil. The obtained oil is stripped with a solvent selected from acetone, methyl ethyl ketone, methyl isobutyl ketone, ethanol, methanol, isopropanol, n-butanol, ethyl acetate or methyl acetate, preferably acetone to obtain a solid. The solid is then treated with a suitable solvent to obtain a clear solution. This solution is then treated with hydrochloric acid at room temperature to obtain Barnidipine hydrochloride.

Barnidipine hydrochloride obtained according to the process of the present invention is substantially free of other impurities and has chemical purity of more than 99.5% and chiral purity of more than 99.7%, with all known impurities below 0.15% and unknown impurities below 0.1%.

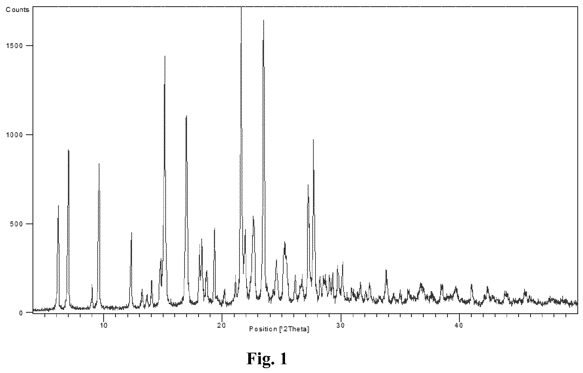

In a preferred embodiment, Barnidipine hydrochloride obtained by the process of the present invention is Form I or amorphous, preferably Form I. Preferably, Barnidipine hydrochloride Form I obtained by the process of the present invention is characterized by X-ray diffraction pattern as shown in Fig. 1. It is further characterized by peaks at 2θ values of about 6.15, 7.01, 9.60, 15.13, 16.97, 21.55, 21.89, 23.47, 27.25 and 27.71± 0.2 degrees.

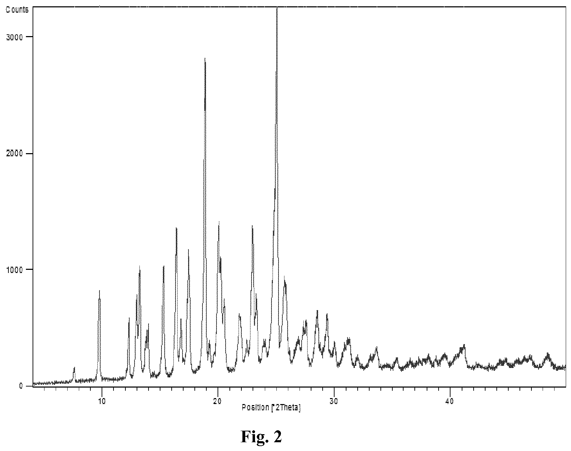

In a preferred embodiment, Barnidipine obtained by the process of the present invention is characterized by X-ray diffraction pattern as shown in Fig. 2. It is further characterized by peaks at 2θ values of about 7.60, 9.78, 12.30, 12.98, 13.24, 13.79, 15.29, 16.41, 16.84, 18.87, 20.03, 22.97 and 25.08 ± 0.2 degrees.

In a preferred embodiment, the process for preparation of Barnidipine or a pharmaceutically acceptable salt thereof, is as shown in Scheme 3 below,

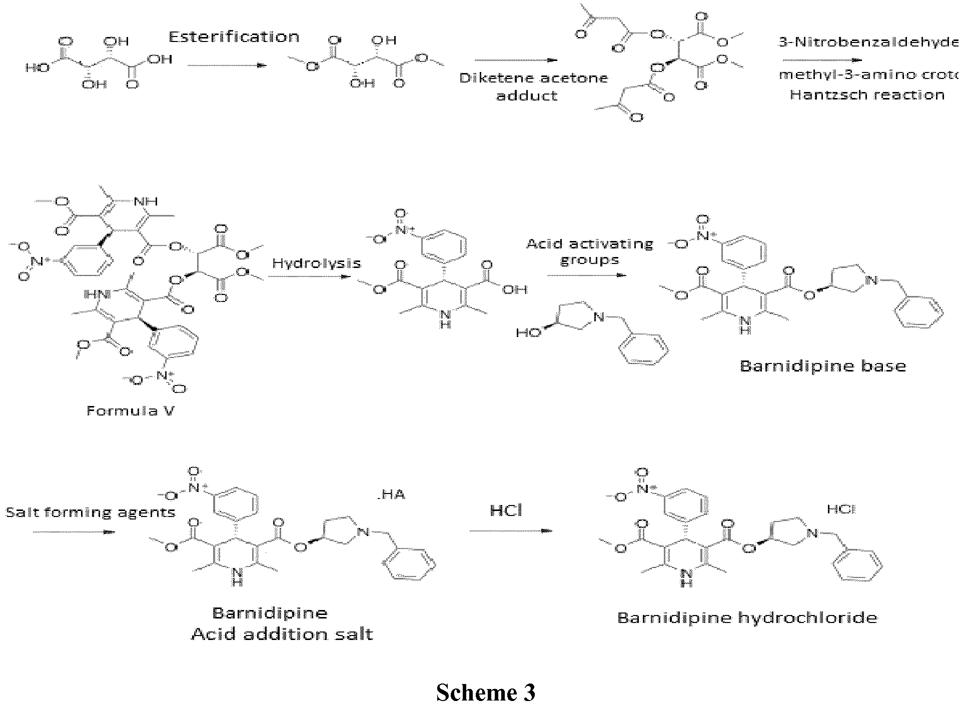

(2S,3S)-dimethyl 2,3-bis(3-oxobutanoyloxy) butanedioate can be prepared by any process known in the art.

In a preferred embodiment, (2S,3S)-dimethyl 2,3-dihydroxysuccinate is treated with a solvent selected from toluene, xylene, pentane, hexane or heptane, preferably toluene followed by addition of 2,2,6-trimethyl-4H-1,3-dioxin-4-one to obtain a reaction mixture. The obtained reaction mixture is refluxed for 5 to 10 hours, preferably for 8 hours. The mixture is cooled to room temperature and further maintained under stirring for 0.5 to 3 hours, preferably for 1 hour. The mixture is then filtered and the filtrate is concentrated to obtain (2S,3S)-dimethyl 2,3-bis(3-oxobutanoyloxy) butanedioate.

(2S,3S)-dimethyl-2,3-dihydroxysuccinate, can be prepared by any process known in the art.

In a preferred embodiment, the process for preparation of (2S,3S)-dimethyl 2,3-dihydroxysuccinate comprises dissolving D-(-)-tartaric acid in a solvent selected from methanol, ethanol, isopropanol, n-propanol or n-butanol, preferably methanol to obtain a solution. The obtained solution is treated with a reagent selected from thionyl chloride, phosphorus pentachloride or phosphorus trichloride, preferably thionyl chloride and the mixture is refluxed for 3 to 7 hours, preferably for 5 hours to obtain a reaction mixture. The obtained reaction mixture is cooled to room temperature, preferably to 30°C, followed by quenching of reaction mixture using a base such as potassium carbonate. The reaction mixture is maintained under stirring and filtered. The filtrate is concentrated to obtain an oily residue. The oily residue is treated with a solvent selected from dichloromethane, dichloroethane, chloroform or carbon tetrachloride, preferably dichloromethane. The mixture is maintained under stirring for 0.5 to 2 hours, preferably for 1 hour and filtered. The filtrate is concentrated to obtain (2S,3S)-dimethyl-2,3-dihydroxysuccinate as an oil.

D-(-)-tartaric acid and 2,2,6-trimethyl-4H-1,3-dioxin-4-one are prepared by the processes known in the art.

A more preferred embodiment of the present invention provides a process for preparation of (R)-5-(methoxycarbonyl)-2,6-dimethyl-4-(3-nitrophenyl)-1,4-dihydropyridine-3-carboxylic acid using (2S,3S)-dimethyl-2,3-bis[(4S)-5-methoxycarbonyl-2,6-dimethyl-4-(3-nitrophenyl)-1,4-dihydropyridine-3-carboxy] butanedioate.

Furthermore, the specification describes a pharmaceutical composition comprising Barnidipine hydrochloride or Barnidipine malate, prepared by the process of the present invention. Barnidipine hydrochloride or Barnidipine malate obtained by the process of the present invention may be combined with pharmaceutically acceptable excipients to obtain suitable pharmaceutical compositions, used in the treatment of mild to moderate hypertension.

Unless otherwise indicated, the following definitions are set forth to illustrate and define the meaning and scope of the various terms used to describe the invention herein.

The term "substantially free" means Barnidipine or pharmaceutically acceptable salt thereof, in particular Barnidipine hydrochloride or Barnidipine malate having less than about 1%, preferably less than about 0.5%, more preferably less than about 0.3%, most preferably less than about 0.15% of impurities, including other polymorphic forms.

The term "reflux temperature" means the temperature at which the solvent or solvent system refluxes or boils at atmospheric pressure.

The term 'room temperature' means the temperature in the range of 20 to 40°C, preferably 25 to 30°C.

The term "pharmaceutically acceptable" means that which is useful in preparing a pharmaceutical composition that is generally non-toxic and is not biologically undesirable and includes that which is acceptable for human pharmaceutical use.

X-ray powder diffraction pattern was obtained on X'Pert PRO, PANalytical, diffractometer equipped with X'Celerator detector using Copper Kα (n = 1.5406 A°) radiation with scanning range between 2-theta 4°-50° at a scanning speed of 6.27°/min.

The following examples are for illustrative purposes only and are not intended to limit the scope of the invention in any way.

### Examples:

### Example 1: Preparation of (2S,3S)-dimethyl-2,3-dihydroxysuccinate

To a solution of D-(-)-tartaric acid (1 kg, 6.66 mol) in methanol (10 L) was slowly added thionyl chloride (0.1kg, 0.84 mol) at 25 to 30°C. The mixture was heated to reflux for 5 hours and then cooled to 30°C. To this mixture was added potassium carbonate (0.24 Kg, 1.74 mol) at the same temperature. The mixture was then maintained at the same temperature under stirring and then filtered. The filtrate was concentrated to obtain an oil. To this oil, was added dichloromethane (3L) to obtain a solution which was maintained under stirring for 1 hour. The solution was filtered and the filtrate was subjected to distillation to obtain the titled product as a colorless oil. Yield: 1.08 Kg (91%)

### Example 2:

### A: Preparation of (2S,3S)-dimethyl 2,3-bis (3-oxobutanoyloxy) butanedioate

(2S,3S)-dimethyl-2,3-dihydroxysuccinate (1.07Kg, 6.0 mol) was taken in toluene (8 L) to obtain a mixture. 2,2,6-trimethyl-4H-1,3-dioxin-4-one (1.88 Kg, 13.22 mol) was added to the above mixture and the obtained reaction mixture was refluxed for 8 hours. The mixture was cooled to 25 to 30°C and maintained under stirring at the same temperature for 1 hour. The mixture was filtered and the filtrate was concentrated to obtain the titled product as an oil. Yield: 1.97 Kg (95%)

Similarly, (2S,3S)-diethyl 2,3-bis(3-oxobutanoyloxy) butanedioate is prepared using (2S,3S)-diethyl-2,3-dihydroxysuccinate.

### B: Preparation of (1R,2S,5R)-2-isopropyl-5-methyl-1-(3-oxobutanoyloxy) cyclohexane

(1R,2S,5R)-2-isopropyl-5-methyl-cyclohexan-1-ol is reacted with 2,2,6-trimethyl-4H-1,3-dioxin-4-one as per the process described in Example 2A to obtain the titled compound.

### C: Preparation of (3S)-(acetoacetoxy)-1-benzylpyrrolidine

(S)-1-benzyl-3-hydroxypyrrolidine is reacted with 2,2,6-trimethyl-4H-1,3-dioxin-4-one as per the process described in Example 2A to obtain the titled compound.

### D: Preparation of (S)-alpha (acetoacetoxy) phenyl acetic acid methyl ester

(S)-2-hydroxy-2-phenyl acetic acid methyl ester is reacted with 2,2,6-trimethyl-4H-1,3-dioxin-4-one as per the process described in Example 2A to obtain the titled compound.

Similarly, (S)-alpha (acetoacetoxy) phenyl acetic acid ethyl ester is prepared using (S)-2-hydroxy-2-phenyl acetic acid ethyl ester.

### E: Preparation of Dimethyl-(S)-alpha (acetoacetoxy)-butanedioate

Dimethyl-(S)-2-hydroxybutanedioate is reacted with 2,2,6-trimethyl-4H-1,3-dioxin-4-one as per the process described in Example 2A to obtain the titled compound. Similarly, Diethyl-(S)-alpha(acetoacetoxy)-butanedioate is prepared using Diethyl-(S)-2-hydroxybutanedioate.

### Example 3:

### A: Preparation of (2S,3S)-dimethyl-2,3-bis[(4S)-5-methoxycarbonyl-2,6-dimethyl-4-(3-nitrophenyl)-1,4-dihydropyridine-3-carboxy] butanedioate

In methanol (9.54 L) were taken, (2S,3S)-dimethyl 2,3-bis (3-oxobutanoyloxy) butanedioate (1.91Kg, 5.51mol), m-nitrobenzaldehyde (1.75Kg, 11.57 mol), methyl-3-amino crotonate (1.52 Kg, 13.21 mol) and ammonium acetate (0.191Kg, 2.47 mol) to obtain a mixture. The reaction mixture was refluxed for 5-6 hours followed by cooling to 10 to 15°C and was further maintained at the same temperature under stirring for 1 hour. The reaction mixture was filtered and the wet cake obtained was treated with methanol (3.0 L) to obtain a slurry. The slurry was filtered and the solid obtained was washed with methanol (1200 ml) and dried at 55-60°C to yield the titled product as a solid. Yield: 0.65 Kg (14.5%); Chemical Purity (HPLC): more than 96% Similarly, (2S,3S)-diethyl-2,3-bis[(4S)-5-methoxycarbonyl-2,6-dimethyl-4-(3-nitro phenyl)-1,4-dihydropyridine-3-carboxy]butanedioate is prepared using (2S,3S)-diethyl 2,3-bis(3-oxobutanoyloxy) butanedioate.

### B: Preparation of (1R,2S,5R)-2-isopropyl-5-methyl-1-[(4S)-5-methoxycarbonyl-2,6-dimethyl-4-(3-nitrophenyl)-1,4-dihydropyridine-3-carboxy] cyclohexane

(1R,2S,5R)-2-isopropyl-5-methyl-1-(3-oxobutanoyloxy)cyclohexane is reacted with m-nitrobenzaldehyde and methyl-3-amino crotonate as per the process described in Example 3A to obtain the titled compound.

### C: Preparation of (3'S,4S)-1-benzyl-3-pyrrolidinyl-methyl-1,4-dihydro-2,6-dimethyl-4-(3-nitrophenyl)-3,5-pyridine dicarboxylate

(3S)-(acetoacetoxy)-1-benzylpyrrolidine is reacted with m-nitrobenzaldehyde and methyl-3-amino crotonate as per the process described in Example 3A to obtain the titled compound.

### D: Preparation of (S)-alpha [(4S)-5-methoxycarbonyl-2,6-dimethyl-4-(3-nitrophenyl)-1,4-dihydropyridine-3-carboxy] phenyl acetic acid methyl ester

(S)-alpha (acetoacetoxy) phenyl acetic acid methyl ester is reacted with m-nitrobenzaldehyde and methyl-3-amino crotonate as per the process described in Example 3A to obtain the titled compound.

Similarly, (S)-alpha [(4S)-5-methoxycarbonyl-2,6-dimethyl-4-(3-nitrophenyl)-1,4-dihydropyridine-3-carboxy] phenyl acetic acid ethyl ester is prepared using (S)-alpha (acetoacetoxy) phenyl acetic acid ethyl ester.

### E: Preparation of Dimethyl-(S)-alpha [(4S)-5-methoxycarbonyl-2,6-dimethyl-4-(3-nitrophenyl)-1,4-dihydropyridine-3-carboxy] butanedioate

Dimethyl-(S)-alpha (acetoacetoxy)-butanedioate is reacted with m-nitrobenzaldehyde and methyl-3-amino crotonate as per the process described in Example 3A to obtain the titled compound.

Similarly, Diethyl-(S)-alpha[(4S)-5-methoxycarbonyl-2,6-dimethyl-4-(3-nitro phenyl) -1,4-dihydropyridine-3-carboxy] butanedioate is prepared using Diethyl-(S)-alpha (acetoacetoxy)-butanedioate.

### Example 4: Preparation of (R)-5-(methoxycarbonyl)-2,6-dimethyl-4-(3-nitro phenyl)-1,4-dihydropyridine-3-carboxylic acid

A mixture of (2S,3S)-dimethyl-2,3-bis[(4S)-5-methoxycarbonyl-2,6-dimethyl-4-(3-nitrophenyl)-1,4-dihydropyridine-3-carboxy] butanedioate (0.2Kg, 0.248 mol) and potassium hydroxide (85%) (0.049Kg, 0.873 mol) in ethylene glycol (0.6L) was heated to 60 to 70°C for 3 hours. The reaction mixture was cooled to 25 to 30°C followed by addition of water (2L). The mixture was treated with dichloromethane (3x1L) and was then subjected to layer separation. The pH of aqueous layer was adjusted to 4 to 5 by addition of acetic acid (43 ml). The obtained precipitate was filtered, washed with water (2L) and dried at 50-55°C to obtain the titled product as solid. Yield: 0.121 Kg (73%);
Chemical purity: more than 96 %; Chiral purity: more than 99 %
Alternatively, a compound selected from (2S,3S)-diethyl-2,3-bis[(4S)-5-methoxy carbonyl-2,6-dimethyl- 4-(3-nitrophenyl)- 1,4-dihydropyridine- 3-carboxy] butanedioate, (1R,2S,5R)-2-isopropyl-5-methyl-1-[(4S)-5-methoxycarbonyl -2,6-dimethyl-4-(3-nitrophenyl)-1,4-dihydropyridine-3-carboxy]cyclohexane, (S)-alpha [(4S)-5-methoxycarbonyl- 2,6-dimethyl-4-(3-nitrophenyl)- 1,4-dihydropyridine-3-carboxy]phenyl acetic acid methyl ester, (S)-alpha [(4S)-5-methoxycarbonyl-2,6-dimethyl-4-(3-nitrophenyl)-1,4-dihydro pyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy] butanedioate or Diethyl-(S)-alpha [(4S)-5-methoxy carbonyl-2,6-dimethyl- 4-(3-nitrophenyl)- 1,4-dihydropyridine- 3-carboxy] butanedioate is subjected to hydrolysis using the reaction conditions as described in Example 4 to obtain the titled compound.

### Example 5: Preparation of (3'S,4S)-1-benzyl-3-pyrrolidinyl-methyl-1,4-dihydro-2,6-dimethyl-4-(3-nitrophenyl)-3,5-pyridine dicarboxylate

### Method I:

(R)-5-(methoxycarbonyl)-2,6-dimethyl-4-(3-nitrophenyl)-1,4-dihydropyridine-3-carboxylic acid (5.0g, 0.015 mol) and dimethyl formamide (6.8ml) were taken in methylene chloride (28ml) to obtain a mixture. To this mixture was added thionyl chloride (1.85g, 0.016 mol) at -10 to 0°C and the mixture was stirred for 2 hours. A solution of (S)-1-benzyl-3-hydroxypyrrolidine (2.78 g, 0.016 mol) in methylene chloride (17 ml) was added to the mixture in a dropwise manner at -20 to -10°C. The reaction mixture was stirred for 3 hours and then quenched with water (57 ml). The layers were separated. The organic layer was washed with 10% sodium bicarbonate solution (2x18 ml) followed by water (57 ml). The organic layer was dried over sodium sulphate and then distilled to obtain the titled product as an oil. Yield: 6.8g (92%)

### Method II:

(R)-5-(methoxycarbonyl)-2,6-dimethyl-4-(3-nitrophenyl)-1,4-dihydropyridine-3-carboxylic acid (25g, 0.075mol), N,N'-dicyclohexylcarbodiimide (15.53g, 0.075 mol), 4-dimethylaminopyridine (1.75g, 0.014 mol) and (S)-1-benzyl-3-hydroxypyrrolidine (13.61g, 0.077 mol) were taken in toluene (250 ml) to obtain a mixture. The mixture was refluxed for 3 hours. The mixture was then cooled to 25 to 30°C and was maintained at the same temperature under stirring for 3 hours. The mixture was filtered and the filtrate was washed with water (150 ml), 5% sodium bicarbonate solution (150 ml) and water (150 ml), dried over sodium sulfate and distilled to obtain the titled product as an oil. Yield: 36.6g (99%)

### Example 6: Preparation of (3'S,4S)-1-benzyl-3-pyrrolidinyl-methyl-1,4-dihydro-2,6-dimethyl-4-(3-nitrophenyl)-3,5-pyridinedicarboxylate malate

(3'S,4S)-1-benzyl-3-pyrrolidinyl-methyl-1,4-dihydro-2,6-dimethyl-4-(3-nitro phenyl)-3,5-pyridinedicarboxylate (36.6 g, 0.074 mol) was dissolved in acetone (230 ml) to obtain a solution. To this solution, was added L-malic acid (9.97 g, 0.074 mol) and the obtained mixture was maintained under stirring for about 4 to 8 hours at 25 to 30°C followed by cooling to 0 to 5°C. The mixture was maintained under stirring at the same temperature for 1 to 2 hours. The mixture was filtered and the product obtained was dried at 55-60°C to get titled product as a solid. Yield: 37.5g (80%) Chemical Purity (HPLC): more than 98 %; Chiral Purity (HPLC): more than 98 %.

### Example 7:

**a) Purification of (3'S,4S)-1-benzyl-3-pyrrolidinyl-methyl-1,4-dihydro-2,6-dimethyl-4-(3-nitrophenyl)-3,5-pyridinedicarboxylate malate**
   (3'S,4S)-1-benzyl-3-pyrrolidinyl-methyl-1,4-dihydro-2,6-dimethyl-4-(3-nitro phenyl)-3,5-pyridinedicarboxylate malate (35g, 0.056mol) was treated with methanol (560 ml) to obtain a suspension. The suspension was heated at 60 to 70°C to obtain a clear solution. The solution was concentrated at atmospheric pressure to reduce the volume and the obtained mixture was stirred for about 7 to 8 hours at 25 to 30°C. The precipitate obtained was filtered, washed with methanol (2x20 ml) and dried at 55 to 60°C to yield pure titled product as a solid. Yield: 28.8g (82%)
   Chemical Purity (HPLC): more than 99 %; Chiral Purity (HPLC): more than 99%.
b) (3'S,4S)-1-benzyl-3-pyrrolidinyl- methyl-1,4-dihydro -2,6-dimethyl- 4-(3-nitro phenyl)-3,5-pyridinedicarboxylate malate obtained from Example 7a (27g, 0.043mol) was treated with methanol (486 ml) to obtain a suspension. The suspension was heated at 60 to 70°C to obtain a clear solution. The solution was concentrated at atmospheric pressure to reduce the volume and the obtained mixture was stirred for about 7 to 8 hours at 25 to 30°C. The precipitate obtained was filtered, washed with methanol (2x15 ml) and dried at 55 to 60°C to yield pure titled product as a solid. Yield: 24 g (89%)
   Chemical Purity (HPLC): more than 99.2%; Chiral Purity (HPLC): more than 99.2 %.
c) (3'S,4S)-1-benzyl-3-pyrrolidinyl- methyl-1,4-dihydro- 2,6-dimethyl -4-(3-nitro phenyl)-3,5-pyridinedicarboxylate malate obtained from Example 7b (23g, 0.037mol) was treated with methanol (414 ml) to obtain a suspension. The suspension was heated at 60 to 70°C to obtain a clear solution. The solution was treated with activated charcoal (1.25g) followed by stirring the mixture for ½ hour at 60 to 70°C. The mixture was then filtered through a hyflobed. The bed was then washed with hot methanol (23 ml). The filtrate was concentrated at atmospheric pressure to reduce the volume. The obtained mixture was stirred for 7 to 8 hours at 25 to 30°C. The precipitate obtained was filtered, washed with methanol (2x20 ml) and dried at 55 to 60°C to yield pure titled product as a solid. Yield: 20.4g (89%)
   Chemical Purity (HPLC): more than 99.4%; Chiral Purity (HPLC): more than 99.5 %.

### Example 8: Preparation of (3'S,4S)-1-benzyl-3-pyrrolidinyl-methyl-1,4-dihydro-2,6-dimethyl-4-(3-nitrophenyl)-3,5-pyridinedicarboxylate hydrochloride

(3'S,4S)-1-benzyl-3-pyrrolidinyl-methyl-1,4-dihydro-2,6-dimethyl-4-(3-nitro phenyl)-3,5-pyridinedicarboxylate malate (17g, 0.027 mol) was taken in dichloromethane (102 ml) to obtain a suspension. The suspension was treated with 6.5% sodium bicarbonate solution (71 ml) to obtain a mixture and the mixture was stirred for ½ hour at 25 to 30°C. The mixture was subjected to layer separation. The organic layer was washed with water (85 ml) and then concentrated to get an oil. The oily substance was stripped with acetone (17ml) to obtain a solid. The solid obtained was dissolved in acetone (173 ml) to get a clear solution, filtered and washed with acetone (27 ml). To this solution was added conc. HCl (35%) (3.4 gm, 0.033 mol) in a dropwise manner and the mixture was stirred for 2 hours at 25 to 30°C. The precipitate obtained was filtered, washed with acetone (2x17 ml) and dried to get the titled product as a solid. Yield: 12.6g (88%)
Chemical Purity(HPLC): more than 99.5 %; Chiral Purity (HPLC): more than 99.5 %.

## Claims

1. A process for preparation of Barnidipine or a pharmaceutically acceptable salt thereof, comprising the steps of,
a) providing a chiral compound of Formula II, 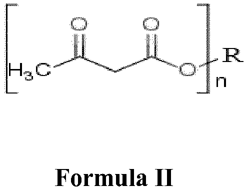 where R is a chiral auxiliary moiety; and n = 1 - 4;
b) treating said chiral compound of Formula II with m-nitrobenzaldehyde and methyl-3-aminocrotonate under Hantzsch reaction conditions to obtain a chiral compound of Formula III; 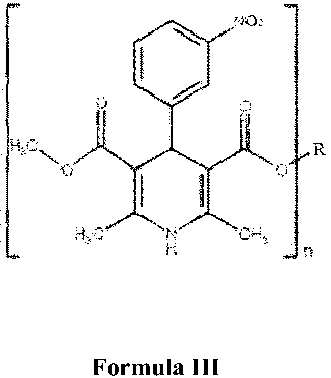 where R is a chiral auxiliary moiety; and n = 1 - 4; and
c) converting said chiral compound of Formula III to Barnidipine or a pharmaceutically acceptable salt thereof, wherein said
chiral auxiliary moiety is selected from the group consisting of (2S,3S)-disubstituted butanedioate, (1R,2S,5R)-2-isopropyl- 5-methyl- cyclohexyl, N-benzyl- 3(S)-pyrrolidinyl, (S)-alpha-substituted phenyl acetic acid ester and (S)-alpha-substituted butanedioate.

2. The process as claimed in claim 1, wherein said chiral compound of Formula II is prepared by reacting a chiral auxiliary reactant with diketene, acetoacetic acid or a derivative thereof.

3. The process as claimed in claim 2, wherein said chiral auxiliary reactant is selected from 2S,3S-dihydroxybutanedioic acid, 2S,3S-dihydroxybutanedioic acid ester, (1R,2S,5R)-2-isopropyl-5-methyl-cyclohexanol, N-benzyl-3(S)-hydroxy pyrrolidine, (S)-alpha hydroxy phenyl acetic acid, (S)-alpha hydroxy phenyl acetic acid ester, (S)-alpha hydroxy butanedioic acid or (S)-alpha hydroxy butanedioic acid ester and said derivative of acetoacetic acid is selected from acetoacetic acid ester or 2,2,6-trimethyl-4H-1,3-dioxin-4-one.

4. The process as claimed in claim 1, wherein said compound of Formula II is selected from the group consisting of (2S,3S)-dimethyl 2,3-bis(3-oxobutanoyloxy) butanedioate, (2S,3S)-diethyl 2,3-bis(3-oxobutanoyloxy) butanedioate, (1R,2S,5R)-2-isopropyl- 5-methyl- 1-(3-oxobutanoyloxy)cyclohexane, (3S)-(acetoacetoxy)-1-benzylpyrrolidine, (S)-alpha (acetoacetoxy) phenyl acetic acid methyl ester, (S)-alpha (acetoacetoxy) phenyl acetic acid ethyl ester, Dimethyl-(S)-alpha(acetoacetoxy)-butanedioate and Diethyl-(S)-alpha (acetoacetoxy)-butanedioate and said compound of Formula III is selected from the group consisting of (2S,3S)-dimethyl-2,3-bis[(4S)-5-methoxycarbonyl-2,6-dimethyl-4-(3-nitrophenyl)-1,4-dihydropyridine-3-carboxy] butanedioate, (2S,3S)-diethyl-2,3-bis[(4S)-5-methoxycarbonyl-2,6-dimethyl-4-(3-nitrophenyl)-1,4-dihydropyridine-3-carboxy]butanedioate, (1R,2S,5R)-2-isopropyl-5-methyl-1-[(4S)-5-methoxy carbonyl-2,6-dimethyl- 4-(3-nitrophenyl)- 1,4-dihydropyridine-3-carboxy] cyclohexane, (3'S,4S)-1-benzyl-3-pyrrolidinyl-methyl-1,4-dihydro-2,6-dimethyl-4-(3-nitrophenyl)-3,5-pyridine dicarboxylate, (S)-alpha [(4S)-5-methoxycarbonyl-2,6-dimethyl-4-(3-nitrophenyl)-1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydropyridine-3-carboxy] butanedioate and Diethyl-(S)-alpha [(4S)-5-methoxycarbonyl-2,6-dimethyl-4-(3-nitrophenyl)-1,4-dihydropyridine-3-carboxy] butanedioate.

5. The process as claimed in claim 1, wherein said Hantzsch reaction is carried out in the presence of a solvent selected from methanol, ethanol, n-propanol, isopropanol, n-butanol, toluene, acetonitrile, DMF, cyclohexane or ethylene glycol.

6. The process as claimed in claim 1, wherein said chiral compound of Formula III is converted to Barnidipine or a pharmaceutically acceptable salt thereof by a process comprising the steps of,
a) subjecting said chiral compound of Formula III to selective hydrolysis to obtain (R)-5-(methoxycarbonyl)- 2,6-dimethyl- 4-(3-nitrophenyl)-1,4-dihydropyridine-3-carboxylic acid; and
b) converting said (R)-5-(methoxycarbonyl)-2,6-dimethyl-4-(3-nitro phenyl)-1,4-dihydropyridine-3-carboxylic acid to Barnidipine or a pharmaceutically acceptable salt thereof,
wherein said chiral compound of Formula III is selected from the group consisting of (2S,3S)-dimethyl-2,3-bis[(4S)-5-methoxycarbonyl-2,6-dimethyl-4-(3-nitrophenyl)-1,4-dihydropyridine-3-carboxy] butanedioate, (2S,3S)-diethyl-2,3-bis[(4S)-5-methoxycarbonyl-2,6-dimethyl-4-(3-nitrophenyl)-1,4-dihydropyridine-3-carboxy] butanedioate, (1R,2S,5R)-2-isopropyl-5-methyl-1-[(4S)-5-methoxycarbonyl-2,6-dimethyl- 4-(3-nitrophenyl)-1,4-dihydropyridine-3-carboxy]cyclohexane, (S)-alpha [(4S)-5-methoxycarbonyl-2,6-dimethyl-4-(3-nitrophenyl)-1,4-dihydropyridine-3-carboxy] phenyl acetic acid methyl ester, (S)-alpha [(4S)-5-methoxycarbonyl-2,6-dimethyl-4-(3-nitrophenyl)-1,4-dihydropyridine-3-carboxy] phenyl acetic acid ethyl ester, Dimethyl-(S)-alpha [(4S)-5-methoxycarbonyl-2,6-dimethyl-4-(3-nitrophenyl)-1,4-dihydro pyridine-3-carboxy] butanedioate and Diethyl-(S)-alpha [(4S)-5-methoxy carbonyl-2,6-dimethyl -4-(3-nitrophenyl) -1,4-dihydropyridine-3-carboxy]butanedioate.

7. The process as claimed in claim 6, wherein said selective hydrolysis in step a) is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxyethane, polyethylene glycol or water.

8. The process as claimed in claim 7, wherein said selective hydrolysis is carried out in the presence of ethylene glycol at a temperature of about 50° to 70° C.

9. A process for preparation of Barnidipine or a pharmaceutically acceptable salt thereof comprising the steps of, 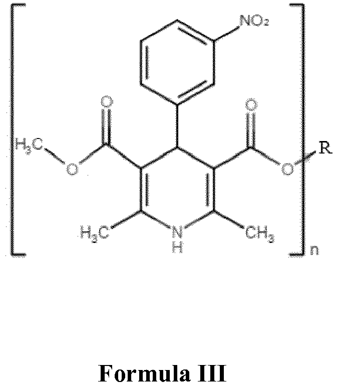
a) providing a chiral compound of Formula III, where R is a chiral auxiliary moiety; and n = 1 - 4;
b) subjecting said chiral compound of Formula III to selective hydrolysis to obtain (R)-5-(methoxycarbonyl)- 2,6-dimethyl- 4-(3-nitrophenyl)-1,4-dihydropyridine-3-carboxylic acid; and
c) converting said (R)-5-(methoxycarbonyl)-2,6-dimethyl-4-(3-nitrophenyl)-1,4-dihydropyridine-3-carboxylic acid to Barnidipine or a pharmaceutically acceptable salt thereof,
wherein said chiral compound of Formula III is selected from (2S,3S)-dimethyl-2,3-bis[(4S)-5-methoxycarbonyl-2,6-dimethyl-4-(3-nitrophenyl)-1,4-dihydropyridine-3-carboxy]butanedioate, (2S,3S)-diethyl-2,3-bis[(4S)-5-methoxycarbonyl-2,6-dimethyl-4-(3-nitrophenyl)- 1,4-dihydropyridine-3-carboxy]butanedioate, (1R,2S,5R)-2-isopropyl- 5-methyl- 1-[(4S)-5-methoxycarbonyl-2,6-dimethyl -4-(3-nitrophenyl)-1,4-dihydropyridine-3-carboxy]cyclohexane, (S)-alpha [(4S)-5-methoxycarbonyl-2,6-dimethyl-4-(3-nitrophenyl)-1,4-dihydropyridine-3-carboxy] phenyl acetic acid methyl ester, (S)-alpha [(4S)-5-methoxycarbonyl- 2,6-dimethyl- 4-(3-nitrophenyl)-1,4-dihydropyridine-3-carboxy] phenyl acetic acid ethyl ester, Dimethyl-(S)-alpha [(4S)-5-methoxycarbonyl- 2,6-dimethyl- 4-(3-nitrophenyl)-1,4-dihydropyridine-3-carboxy]butanedioate or Diethyl-(S)-alpha [(4S)-5-methoxycarbonyl-2,6-dimethyl -4-(3-nitrophenyl)- 1,4-dihydropyridine-3-carboxy]butanedioate.

10. The process as claimed in claim 9, wherein said selective hydrolysis is carried out in presence of a base selected from potassium hydroxide, sodium hydroxide, lithium hydroxide, calcium hydroxide or cesium hydroxide and a solvent selected from ethylene glycol, 1,3-propanediol, 1,4-butanediol, tert-butanol, n-butanol, isopropyl alcohol, ethanol, methanol, n-propanol, n-pentanol, isoamyl alcohol, acetone, tetrahydrofuran, dimethoxyethane, polyethylene glycol or water.

11. The process as claimed in claim 6 or claim 10, wherein said (R)-5-(methoxycarbonyl)-2,6-dimethyl- 4-(3-nitrophenyl)- 1,4-dihydropyridine-3-carboxylic acid is converted to Barnidipine or a pharmaceutically acceptable salt thereof, comprising the steps of,
a) coupling (R)-5-(methoxycarbonyl)-2,6-dimethyl- 4-(3-nitrophenyl)- 1,4-dihydropyridine-3-carboxylic acid with (S)-1-benzyl-3-hydroxypyrrolidine in presence of an acid activating agent selected from thionyl chloride, phosphorus pentachloride, oxalyl chloride, pivaloyl chloride, N,N'-dicyclohexylcarbodiimide(DCC), 1,1'-carbonyldiimidazole(CDI) or 3-(ethyliminomethyleneamino)-N,N-dimethylpropan-1-amine(EDC) to obtain Barnidipine;
b) optionally converting said Barnidipine to a pharmaceutically acceptable salt thereof.

12. The process as claimed in claim 11, wherein said Barnidipine obtained in step a) is purified by a process comprising the steps of,
a) treating Barnidipine with a salt forming agent to obtain Barnidipine acid addition salt;
b) purifying said Barnidipine acid addition salt; and
c) converting pure Barnidipine acid addition salt to Barnidipine,
wherein said salt forming agent is a chiral resolving agent.

13. The process as claimed in claim 12, wherein said chiral resolving agent is selected from L-malic acid, D-malic acid, L-tartaric acid, D-tartaric acid, Dibenzoyl-L-tartaric acid, Dibenzoyl-D-tartaric acid, Di-p-toluoyl-L-tartaric acid or Di-p-toluoyl-D-tartaric acid.

14. A process for preparation of (R)-5-(methoxycarbonyl)-2,6-dimethyl-4-(3-nitrophenyl)-1,4-dihydropyridine-3-carboxylic acid comprising the steps of,
a) providing a chiral compound of Formula II or Formula III, 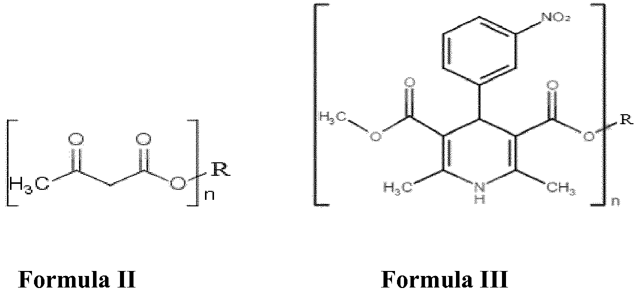 where R is a chiral auxiliary moiety; and n = 1 - 4; and
b) converting said chiral compound of Formula II or Formula III to (R)-5-(methoxycarbonyl)-2,6-dimethyl-4-(3-nitrophenyl)-1,4-dihydropyridine-3-carboxylic acid, wherein said chiral auxiliary moiety is selected from the group consisting of (2S, 3S)-disubstituted butanedioate, (1R,2S,5R)-2-isopropyl-5-methyl-cyclohexyl, N-benzyl-3(S)-pyrrolidinyl, (S)-alpha-substituted phenyl acetic acid ester and (S)-alpha-substituted butanedioate.

15. The process as claimed in claim 14, wherein said (R)-5-(methoxycarbonyl)-2,6-dimethyl-4-(3-nitrophenyl)-1,4-dihydropyridine-3-carboxylic acid is converted to Barnidipine or a pharmaceutically acceptable salt thereof.

16. A chiral compound of Formula III 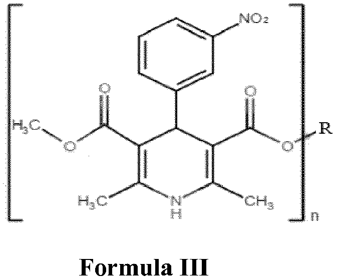 where R is a chiral auxiliary moiety selected from the group consisting of (2S,3S)-disubstituted butanedioate, (1R,2S,5R)-2-isopropyl-5-methyl-cyclohexyl, (S)-alpha-substituted phenyl acetic acid ester and (S)-alpha substituted butanedioate; and n = 1 or 2.

17. The compound as claimed in claim 16, wherein said compound is selected from Formula III A, III B, III D or III E, 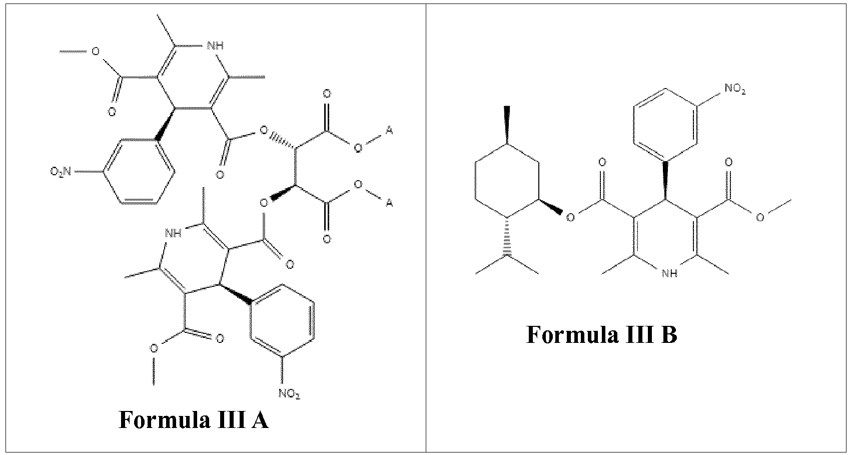 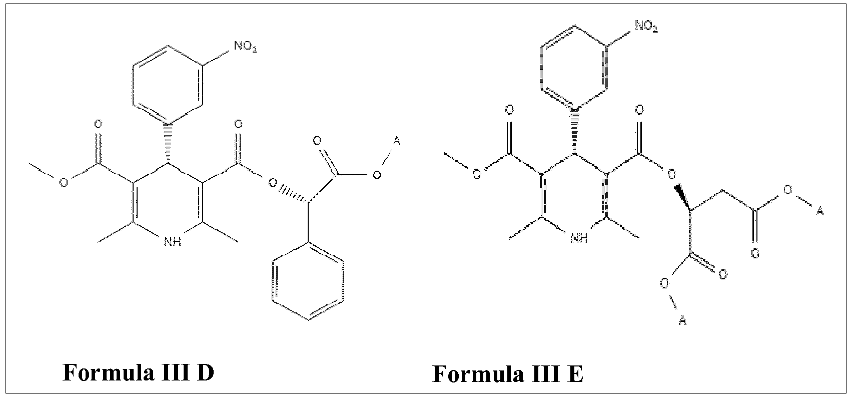 where A is selected from methyl, ethyl, n-propyl, isopropyl or benzyl.

18. Use of a compound of Formula II and/or Formula III in the preparation of Barnidipine or a pharmaceutically acceptable salt thereof.

19. The use as claimed in claim 18, wherein the compound of Formula III is subjected to selective hydrolysis in the presence of ethylene glycol so as to prepare Barnidipine or a pharmaceutically acceptable salt thereof.

## Patentansprüche

1. Verfahren zur Herstellung von Barnidipin oder eines pharmazeutisch akzeptablen Salzes hiervon, wobei das Verfahren die folgenden Stufen umfasst:
a) Bereitstellen einer chiralen Verbindung der Formel II, 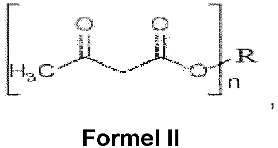 wobei R eine chirale Hilfsgruppe ist und n = 1 bis 4 ist;
b) Behandeln der chiralen Verbindung der Formel II mit m-Nitrobenzaldehyd und Methyl-3-aminocrotonat unter Hantzsch-Reaktionsbedingungen zum Erhalt einer chiralen Verbindung der Formel III, 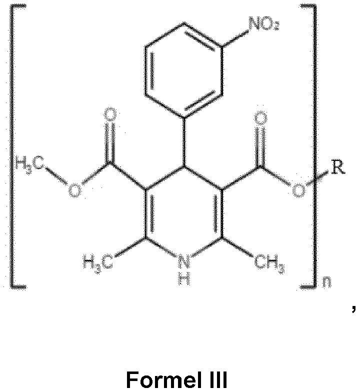 wobei R eine chirale Hilfsgruppe ist und n = 1 bis 4 ist; und
c) Umwandeln der chiralen Verbindung der Formel III in Barnidipin oder ein pharmazeutisch akzeptables Salz hiervon, wobei
die chirale Hilfsgruppe aus der Gruppe ausgewählt ist, die aus (2S,3S)-disubstituiertem Succinat, (1R,2S,5R)-2-Isopropyl-5-methyl-cyclohexyl, N-Benzyl-3(S)-pyrrolidinyl, (S)-alpha-substituiertem Phenylessigsäureester und (S)-alpha-substituiertem Succinat besteht.

2. Verfahren nach Anspruch 1, wobei die chirale Verbindung der Formel II durch Umsetzen eines chiralen Hilfsreaktanden mit Diketen, Acetessigsäure oder einem Derivat hiervon hergestellt wird.

3. Verfahren nach Anspruch 2, wobei der chirale Hilfsreaktand aus 2S,3S-Dihydroxy-bernsteinsäure, 2S,3S-Dihydroxybernsteinsäureester, (1R,2S,5R)-2-Isopropyl-5-methyl-cyclohexanol, N-Benzyl-3(S)-hydroxypyrrolidin, (S)-alpha-Hydroxyphenyl-essigsäure, (S)-alpha-Hydroxyphenylessigsäureester, (S)-alpha-Hydroxybernsteinsäure oder (S)-alpha-Hydroxybernsteinsäureester ausgewählt ist und das Derivat von Acetessigsäure aus Acetessigsäureester oder 2,2,6-Trimethyl-4H-1,3-dioxin-4-on ausgewählt ist.

4. Verfahren nach Anspruch 1, wobei die Verbindung der Formel II aus der Gruppe ausgewählt ist, die aus (2S,3S)-Dimethyl-2,3-bis(3-oxobutanoyloxy)succinat, (2S,3S)-Diethyl-2,3-bis(3-oxobutanoyloxy)succinat, (1R,2S,5R)-2-Isopropyl-5-methyl-1-(3-oxobutanoyloxy)cyclohexan, (3S)-(Acetacetoxy)-1-benzylpyrrolidin, (S)-alpha-(Acetacetoxy)phenylessigsäuremethylester, (S)-alpha-(Acetacetoxy)phenylessigsäureethylester, Dimethyl-(S)-alpha-(acetacetoxy)-succinat und Diethyl-(S)-alpha-(acetacetoxy)-succinat besteht und die Verbindung der Formel III aus der Gruppe ausgewählt ist, die aus (2S,3S)-Dimethyl-2,3-bis[(4S)-5-methoxycarbonyl-2,6-dimethyl-4-(3-nitrophenyl)-1,4-dihydropyridin-3-carboxy]succinat, (2S,3S)-Diethyl-2,3-bis[(4S)-5-methoxycarbonyl-2,6-dimethyl-4-(3-nitrophenyl)-1,4-dihydropyridin-3-carboxy]succinat, (1R,2S,5R)-2-Isopropyl-5-methyl-1-[(4S)-5-methoxycarbonyl-2,6-dimethyl-4-(3-nitrophenyl)-1,4-dihydropyridin-3-carboxy]cyclohexan, (3'S,4S)-1-Benzyl-3-pyrrolidinyl-methyl-1,4-dihydro-2,6-dimethyl-4-(3-nitrophenyl)-3,5-pyridin-dicarboxylat, (S)-alpha-[(4S)-5-Methoxycarbonyl-2,6-dimethyl-4-(3-nitrophenyl)-1,4-dihydropyridin-3-carboxy]phenylessigsäuremethylester, (S)-alpha-[(4S)-5-Methoxycarbonyl-2,6-dimethyl-4-(3-nitrophenyl)-1,4-dihydropyridin-3-carboxy]phenylessigsäureethylester, Dimethyl-(S)-alpha-[(4S)-5-methoxycarbonyl-2,6-dimethyl-4-(3-nitrophenyl)-1,4-dihydropyridin-3-carboxy]succinat und Diethyl-(S)-alpha-[(4S)-5-methoxycarbonyl-2,6-dimethyl-4-(3-nitrophenyl)-1,4-dihydropyridin-3-carboxy]succinat besteht.

5. Verfahren nach Anspruch 1, wobei die Hantzsch-Reaktion in Gegenwart eines Lösemittels durchgeführt wird, das aus Methanol, Ethanol, n-Propanol, Isopropanol, n-Butanol, Toluol, Acetonitril, DMF, Cyclohexan oder Ethylenglycol ausgewählt ist.

6. Verfahren nach Anspruch 1, wobei die chirale Verbindung der Formel III in Barnidipin oder ein pharmazeutisch akzeptables Salz hiervon durch ein Verfahren umgewandelt wird, das die folgenden Stufen umfasst:
a) Unterziehen der chiralen Verbindung der Formel III einer selektiven Hydrolyse zum Erhalt von (R)-5-(Methoxycarbonyl)-2,6-dimethyl-4-(3-nitrophenyl)-1,4-dihydropyridin-3-carbonsäure; und
b) Umwandeln der (R)-5-(Methoxycarbonyl)-2,6-dimethyl-4-(3-nitrophenyl)-1,4-dihydropyridin-3-carbonsäure in Barnidipin oder ein pharmazeutisch akzeptables Salz hiervon,
wobei die chirale Verbindung der Formel III aus der Gruppe ausgewählt ist, die aus (2S,3S)-Dimethyl-2,3-bis[(4S)-5-methoxycarbonyl-2,6-dimethyl-4-(3-nitrophenyl)-1,4-dihydropyridin-3-carboxy]succinat, (2S,3S)-Diethyl-2,3-bis[(4S)-5-methoxycarbonyl-2,6-dimethyl-4-(3-nitrophenyl)-1,4-dihydropyridin-3-carboxy]succinat, (1R,2S,5R)-2-Isopropyl-5-methyl-1-[(4S)-5-methoxycarbonyl-2,6-dimethyl-4-(3-nitrophenyl)-1,4-dihydropyridin-3-carboxy]cyclohexan, (S)-alpha-[(4S)-5-Methoxycarbonyl-2,6-dimethyl-4-(3-nitrophenyl)-1,4-dihydropyridin-3-carboxy]phenylessigsäuremethylester, (S)-alpha-[(4S)-5-methoxycarbonyl-2,6-dimethyl-4-(3-nitrophenyl)-1,4-dihydropyridin-3-carboxy]phenylessigsäureethylester, Dimethyl-(S)-alpha-[(4S)-5-methoxycarbonyl-2,6-dimethyl-4-(3-nitrophenyl)-1,4-dihydropyridin-3-carboxy]succinat und Diethyl-(S)-alpha-[(4S)-5-methoxycarbonyl-2,6-dimethyl-4(-3-nitrophenyl)-1,4-dihydropyridin-3-carboxy]succinat besteht.

7. Verfahren nach Anspruch 6, wobei die selektive Hydrolyse in Stufe a) in Gegenwart einer Base, die aus Kaliumhydroxid, Natriumhydroxid, Lithiumhydroxid, Calciumhydroxid oder Cäsiumhydroxid ausgewählt ist, und eines Lösemittels, das aus Ethylenglycol, 1,3-Propandiol, 1,4-Butandiol, tert.-Butanol, n-Butanol, Isopropylalkohol, Ethanol, Methanol, n-Propanol, n-Pentanol, Isoamylalkohol, Aceton, Tetrahydrofuran, Dimethoxyethan, Polyethylenglycol oder Wasser ausgewählt ist, durchgeführt wird.

8. Verfahren nach Anspruch 7, wobei die selektive Hydrolyse in Gegenwart von Ethylenglycol bei einer Temperatur von etwa 50 °C bis 70 °C durchgeführt wird.

9. Verfahren zur Herstellung von Barnidipin oder eines pharmazeutisch akzeptablen Salzes hiervon, das die folgenden Stufen umfasst:
a) Bereitstellen einer chiralen Verbindung der Formel III 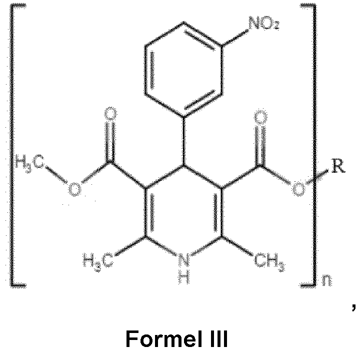 wobei R eine chirale Hilfsgruppe ist und n = 1 bis 4 ist;
b) Unterziehen der chiralen Verbindung der Formel III einer selektiven Hydrolyse zum Erhalt von (R)-5-(Methoxycarbonyl)-2,6-dimethyl-4-(3-nitrophenyl)-1,4-dihydropyridin-3-carbonsäure; und
c) Umwandeln der (R)-5-(Methoxycarbonyl)-2,6-dimethyl-4-(3-nitrophenyl)-1,4-dihydropyridin-3-carbonsäure in Barnidipin oder ein pharmazeutisch akzeptables Salz hiervon,
wobei die chirale Verbindung der Formel III aus (2S,3S)-Dimethyl-2,3-bis[(4S)-5-methoxycarbonyl-2,6-dimethyl-4-(3-nitrophenyl)-1,4-dihydropyridin-3-carboxy]-succinat, (2S,3S)-Diethyl-2,3-bis[(4S)-5-methoxycarbonyl-2,6-dimethyl-4-(3-nitrophenyl)-1,4-dihydropyridin-3-carboxy]succinat, (1R,2S,5R)-2-Isopropyl-5-methyl-1-[(4S)-5-methoxycarbonyl-2,6-dimethyl-4-(3-nitrophenyl)-1,4-dihydropyridin-3-carboxy]cyclohexan, (S)-alpha-[(4S)-5-Methoxycarbonyl-2,6-dimethyl-4-(3-nitrophenyl)-1,4-dihydropyridin-3-carboxy]phenylessigsäuremethylester, (S)-alpha-[(4S)-5-methoxycarbonyl-2,6-dimethyl-4-(3-nitrophenyl)-1,4-dihydropyridin-3-carboxy]phenylessigsäureethylester, Dimethyl-(S)-alpha-[(4S)-5-methoxycarbonyl-2,6-dimethyl-4-(3-nitrophenyl)-1,4-dihydropyridin-3-carboxy]succinat oder Diethyl-(S)-alpha-[(4S)-5-methoxycarbonyl-2,6-dimethyl-4-(-3-nitrophenyl)-1,4-dihydropyridin-3-carboxy]succinat ausgewählt ist.

10. Verfahren nach Anspruch 9, wobei die selektive Hydrolyse in Gegenwart einer Base, die aus Kaliumhydroxid, Natriumhydroxid, Lithiumhydroxid, Calciumhydroxid oder Cäsiumhydroxid ausgewählt ist, und eines Lösemittels, das aus Ethylenglycol, 1,3-Propandiol, 1,4-Butandiol, tert.-Butanol, n-Butanol, Isopropylalkohol, Ethanol, Methanol, n-Propanol, n-Pentanol, Isoamylalkohol, Aceton, Tetrahydrofuran, Dimethoxyethan, Polyethylenglycol oder Wasser ausgewählt ist, durchgeführt wird.

11. Verfahren nach Anspruch 6 oder Anspruch 10, wobei die (R)-5-(Methoxycarbonyl)-2,6-dimethyl-4-(3-nitrophenyl)-1,4-dihydropyridin-3-carbonsäure in Barnidipin oder ein pharmazeutisch akzeptables Salz hiervon umgewandelt wird, wobei das Verfahren die folgenden Stufen umfasst:
a) Kuppeln von (R)-5-(Methoxycarbonyl)-2,6-dimethyl-4-(3-nitrophenyl)-1,4-dihydropyridin-3-carbonsäure mit (S)-1-Benzyl-3-hydroxypyrrolidin in Gegenwart eines säureaktivierenden Mittels, das aus Thionylchlorid, Phosphorpentachlorid, Oxalylchlorid, Pivaloylchlorid, N,N'-Dicyclohexylcarbodiimid (DCC), 1,1'- Carbonyldiimidazol (CDI) oder 3-(Ethylimino-methylenamino)-N,N-dimethylpropan-1-amin (EDC) ausgewählt ist, um Barnidipin zu erhalten;
b) optionales Umwandeln des Barnidipins in ein pharmazeutisch akzeptables Salz hiervon.

12. Verfahren nach Anspruch 11, wobei das in Stufe a) erhaltene Barnidipin mittels eines Verfahrens gereinigt wird, das die folgenden Stufen umfasst:
a) Behandeln von Barnidipin mit einem ein Salz bildenden Mittel zum Erhalt von Barnidipinsäure-Additionssalz;
b) Reinigen des Barnidipinsäure-Additionssalzes; und
c) Umwandeln des reinen Barnidipinsäure-Additionssalzes in Barnidipin,
wobei das ein Salz bildende Mittel ein chirales Trennungsmittel ist.

13. Verfahren nach Anspruch 12, wobei das chirale Trennungsmittel aus L-Äpfelsäure, D-Äpfelsäure, L-Weinsäure, D-Weinsäure, Dibenzoyl-L-weinsäure, Dibenzoyl-D-weinsäure, Di-p-toluoyl-L-weinsäure oder Di-p-toluoyl-D-weinsäure ausgewählt ist.

14. Verfahren zur Herstellung von (R)-5-(Methoxycarbonyl)-2,6-dimethyl-4-(3-nitrophenyl)-1,4-dihydropyridin-3-carbonsäure, das die folgenden Stufen umfasst:
a) Bereitstellen einer chiralen Verbindung der Formel II oder der Formel III 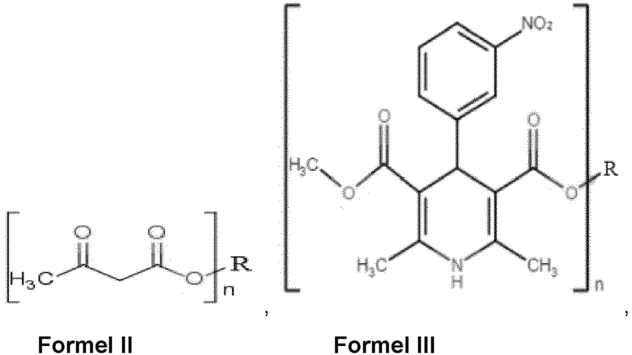 wobei R eine chirale Hilfsgruppe ist und n = 1 bis 4 ist, und
b) Umwandeln der chiralen Verbindung der Formel II oder der Formel III in (R)-5-(Methoxycarbonyl)-2,6-dimethyl-4-(3-nitrophenyl)-1,4-dihydropyridin-3-carbonsäure, wobei die chirale Hilfsgruppe aus der Gruppe ausgewählt ist, die aus (2S,3S)-disubstituiertem Succinat, (1R,2S,5R)-2-Isopropyl-5-methyl-cyclohexyl, N-Benzyl-3(S)-pyrrolidinyl, (S)-alpha-substitutiertem Phenylessigsäureester und (S)-alpha-substituiertem Succinat besteht.

15. Verfahren nach Anspruch 14, wobei die (R)-5-(Methoxycarbonyl)-2,6-dimethyl-4-(3-nitrophenyl)-1,4-dihydropyridin-3-carbonsäure in Barnidipin oder ein pharmazeutisch akzeptables Salz hiervon umgewandelt wird.

16. Chirale Verbindung der Formel III 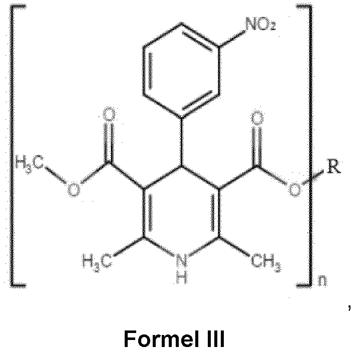 wobei R eine chirale Hilfsgruppe ist, die aus der Gruppe ausgewählt ist, die aus (2S,3S)-disubstituiertem Succinat, (1R,2S,5R)-2-Isopropyl-5-methyl-cyclohexyl, (S)-alpha-substitutiertem Phenylessigsäureester und (S)-alpha-substituiertem Succinat besteht, und wobei n = 1 oder 2 ist.

17. Verbindung nach Anspruch 16, wobei die Verbindung ausgewählt ist aus Formel III A, III B, III D oder III E, 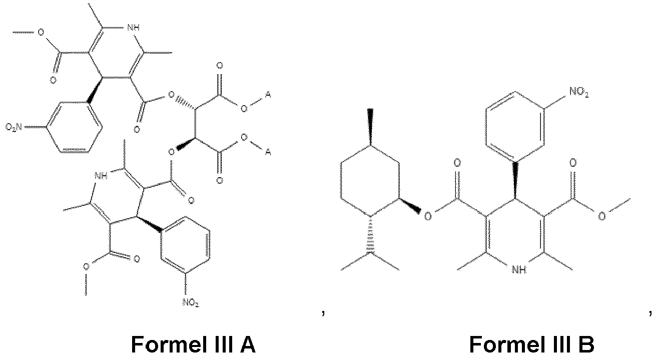 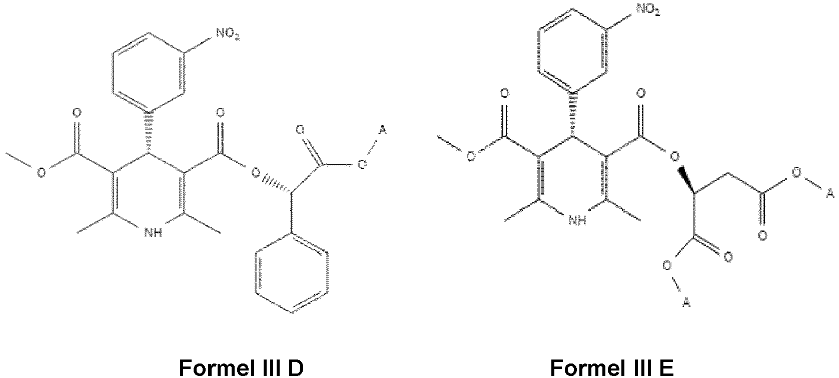 wobei A aus Methyl, Ethyl, n-Propyl, Isopropyl oder Benzyl ausgewählt ist.

18. Verwendung einer Verbindung der Formel II und/oder der Formel III bei der Herstellung von Barnidipin oder eines pharmazeutisch akzeptablen Salzes hiervon.

19. Verwendung nach Anspruch 18, wobei die Verbindung der Formel III einer selektiven Hydrolyse in Gegenwart von Ethylenglycol unterzogen wird, um Barnidipin oder ein pharmazeutisch akzeptables Salz hiervon herzustellen.

## Revendications

1. Procédé de préparation de barnidipine ou d'un sel pharmaceutiquement acceptable de celle-ci, comprenant les étapes de
a) fourniture d'un composé chiral de formule II, 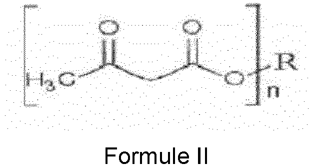 où R est un fragment auxiliaire chiral ; et n = 1 à 4 ;
b) traitement dudit composé chiral de formule II avec du m-nitrobenzaldéhyde et du 3-aminocrotonate de méthyle dans les conditions de la réaction de Hantzsch pour obtenir un composé chiral de formule III : 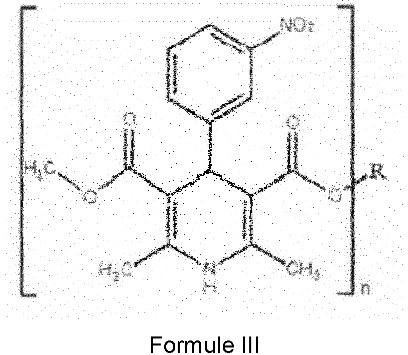 où R est un fragment auxiliaire chiral ; et n = 1 à 4 ; et
c) conversion dudit composé chiral de formule III en barnidipine ou en un sel pharmaceutiquement acceptable de celle-ci, dans lequel ledit fragment auxiliaire chiral est sélectionné dans le groupe constitué par le butanedioate (2S,3S)-disubstitué, le (1R,2S,5R)-2-isopropyl-5-méthyl-cyclohexyle, le N-benzyl-3(S)-pyrrolidinyle, l'ester d'acide phénylacétique (S)-alpha-substitué et le butanedioate (S)-alpha-substitué.

2. Procédé selon la revendication 1, dans lequel ledit composé chiral de formule II est préparé par réaction d'un réactif auxiliaire chirale avec un dicétène, un acide acétoacétique ou un dérivé de celui-ci.

3. Procédé selon la revendication 2, dans lequel ledit réactif auxiliaire chiral est sélectionné parmi l'acide 2S,3S-dihydroxybutanedioïque, l'ester d'acide 2S,3S-dihydroxybutanedioïque, le (1R,2S,5R)-2-isopropyl-5-méthyl-cyclohexanol, la N-benzyl-3(S)-hydroxypyrrolidine, l'acide (S)-alpha-hydroxyphénylacétique, l'ester d'acide (S)-alpha-hydroxyphénylacétique, l'acide (S)-alpha-hydroxybutane-dioïque ou l'ester d'acide (S)-alpha-hydroxybutanedioïque et ledit dérivé de l'acide acétoacétique est sélectionné parmi l'ester d'acide acétoacétique ou la 2,2,6-triméthyl-4H-1,3-dioxin-4-one.

4. Procédé selon la revendication 1, dans lequel ledit composé de formule II est sélectionné dans le groupe constitué par le (2S,3S)-diméthyl-2,3-bis(3-oxobutanoyloxy)butanedioate, le (2S,3S)-diéthyl-2,3-bis(3-oxobutanoyloxy)-butanedioate, le (1R,2S,5R)-2-isopropyl-5-méthyl-1-(3-oxobutanoyloxy)cyclohexane, la (3S)-(acétoacétoxy)-1-benzylpyrrolidine, l'ester méthylique de l'acide (S)-alpha(acétoacétoxy)phénylacétique, l'ester éthylique de l'acide (S)-alpha(acétoacétoxy)phénylacétique, le diméthyl-(S)-alpha(acétoacétoxy)-butanedioate et le diéthyl-(S)-alpha(acétoacétoxy)-butanedioate et ledit composé de formule III est sélectionné dans le groupe constitué par le (2S,3S)-diméthyl-2,3-bis[(4S)-5-méthoxycarbonyl-2,6-diméthyl-4-(3-nitrophényl)-1,4-dihydro-pyridine-3-carboxy]butanedioate, le (2S,3S)-diéthyl-2,3-bis[(4S)-5-méthoxy-carbonyl-2,6-diméthyl-4-(3-nitrophényl)-1,4-dihydropyridine-3-carboxy]butanedioate, le (1R,2S,5R)-2-isopropyl-5-méthyl-1-[(4S)-5-méthoxycarbonyl-2,6-diméthyl-4-(3-nitrophényl)-1,4-dihydropyridine-3-carboxy]cyclohexane, le dicarboxylate de (3'S,4S)-1-benzyl-3-pyrrolidinyl-méthyl-1,4-dihydro-2,6-diméthyl-4-(3-nitrophényl)-3,5-pyridine, l'ester méthylique de l'acide (S)-alpha[(4S)-5-methoxycarbonyl-2,6-dimethyl-4-(3-nitrophényl)-1,4-dihydropyridine-3-carboxy]-phénylique, l'ester éthylique de l'acide (S)-alpha-[(4S)-5-méthoxycarbonyl-2,6-diméthyl-4-(3-nitrophényl)-1,4-dihydropyridine-3-carboxy]phénylacétique, le diméthyl-(S)-alpha[(4S)-5-méthoxycarbonyl-2,6-diméthyl-4-(3-nitrophényl)-1,4-dihydropyridine-3-carboxy]butanedioate et le diéthyl-(S)-alpha[(4S)-5-méthoxy-carbonyl-2,6-diméthyl-4-(3-nitrophényl)-1,4-dihydropyridine-3-carboxy]butanedioate.

5. Procédé selon la revendication 1, dans lequel ladite réaction de Hantzsch est réalisée en présence d'un solvant sélectionné parmi le méthanol, l'éthanol, le n-propanol, l'isopropanol, le n-butanol, le toluène, l'acétonitrile, le DMF, le cyclohexane ou l'éthylène glycol.

6. Procédé selon la revendication 1, dans lequel ledit composé chiral de formule III est converti en barnidipine ou en un sel pharmaceutiquement acceptable de celle-ci par un procédé comprenant les étapes de
a) soumission dudit composé chiral de formule III à une hydrolyse sélective pour obtenir l'acide (R)-5-(méthoxycarbonyl)-2,6-diméthyl-4-(3-nitrophényl)-1,4-dihydropyridine-3-carboxylique ; et
b) conversion dudit acide (R)-5-(méthoxycarbonyl)-2,6-diméthyl-4-(3-nitrophényl)-1,4-dihydropyridine-3-carboxylique en barnidipine ou en un sel pharmaceutiquement acceptable de celle-ci,
dans lequel ledit composé chiral de formule III est sélectionné dans le groupe constitué par le (2S,3S)-diméthyl-2,3-bis[(4S)-5-méthoxycarbonyl-2,6-diméthyl-4-(3-nitrophényl)-1,4-dihydropyridine-3-carboxy]butanedioate, le (2S,3S)-diéthyl-2,3-bis[(4S)-5-méthoxycarbonyl-2,6-diméthyl-4-(3-nitrophényl)-1,4-dihydro-pyridine-3-carboxy]butanedioate, le (1R,2S,5R)-2-isopropyl-5-méthyl-1-[(4S)-5-méthoxycarbonyl-2,6-diméthyl-4-(3-nitrophényl)-1,4-dihydropyridine-3-carboxy]-cyclohexane, l'ester méthylique de l'acide (S)-alpha[(4S)-5-méthoxycarbonyl-2,6-dimethyl-4-(3-nitrophényl)-1,4-dihydropyridine-3-carboxy]phénylique, l'ester éthylique de l'acide (S)-alpha[(4S)-5-méthoxycarbonyl-2,6-diméthyl-4-(3-nitrophényl)-1,4-dihydropyridine-3-carboxy]phénylacétique, le diméthyl-(S)-alpha[(4S)-5-méthoxycarbonyl-2,6-diméthyl-4-(3-nitrophényl)-1,4-dihydro-pyridine-3-carboxy]butanedioate et le diéthyl-(S)-alpha[(4S)-5-méthoxycarbonyl-2,6-diméthyl-4-(3-nitrophényl)-1,4-dihydropyridine-3-carboxy]butanedioate.

7. Procédé selon la revendication 6, dans lequel ladite hydrolyse sélective à l'étape a) est réalisée en présence d'une base sélectionnée parmi l'hydroxyde de potassium, l'hydroxyde de sodium, l'hydroxyde de lithium, l'hydroxyde de calcium ou l'hydroxyde de césium et un solvant sélectionné parmi l'éthylène glycol, le 1,3-propanediol, le 1,4-butanediol, le tert-butanol, le n-butanol, l'alcool isopropylique, l'éthanol, le méthanol, le n-propanol, le n-pentanol, l'alcool isoamylique, l'acétone, le tétrahydrofurane, le diméthoxyéthane, le polyéthylène glycol ou l'eau.

8. Procédé selon la revendication 7, dans lequel ladite hydrolyse sélective est réalisée en présence d'éthylène glycol à une température d'environ 50 à 70 °C.

9. Procédé de préparation de barnidipine ou d'un sel pharmaceutiquement acceptable de celle-ci comprenant les étapes de
a) fourniture d'un composé chiral de formule III 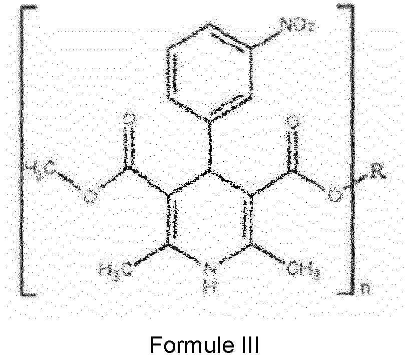 où R est un fragment auxiliaire chiral ; et n = 1 à 4 ;
b) soumission dudit composé chiral de formule III à une hydrolyse sélective pour obtenir l'acide (R)-5-(méthoxycarbonyl)-2,6-diméthyl-4-(3-nitrophényl)-1,4-dihydropyridine-3-carboxylique ; et
c) conversion dudit acide (R)-5-(méthoxycarbonyl)-2,6-diméthyl-4-(3-nitrophényl)-1,4-dihydropyridine-3-carboxylique en barnidipine ou en un sel pharmaceutiquement acceptable de celle-ci,
dans lequel ledit composé chiral de formule III est sélectionné parmi le (2S,3S)-diméthyl-2,3-bis[(4S)-5-méthoxycarbonyl-2,6-diméthyl-4-(3-nitrophényl)-1,4-dihydropyridine-3-carboxy]butanedioate, le (2S,3S)-diéthyl-2,3-bis[(4S)-5-méthoxycarbonyl-2,6-diméthyl-4-(3-nitrophényl)-1,4-dihydropyridine-3-carboxy]-butanedioate, le (1R,2S,5R)-2-isopropyl-5-méthyl-1-[(4S)-5-méthoxycarbonyl-2,6-diméthyl-4-(3-nitrophényl)-1,4-dihydropyridine-3-carboxy]cyclohexane, l'ester méthylique de l'acide (S)-alpha[(4S)-5-méthoxycarbonyl-2,6-dimethyl-4-(3-nitrophényl)-1,4-dihydropyridine-3-carboxy]phénylique, l'ester éthylique de l'acide (S)-alpha[(4S)-5-méthoxycarbonyl-2,6-diméthyl-4-(3-nitrophényl)-1,4-dihydropyridine-3-carboxy]phénylacétique, le diméthyl-(S)-alpha[(4S)-5-méthoxycarbonyl-2,6-diméthyl-4-(3-nitrophényl)-1,4-dihydropyridine-3-carboxy]-butanedioate ou le diéthyl-(S)-alpha[(4S)-5-méthoxycarbonyl-2,6-diméthyl-4-(3-nitrophényl)-1,4-dihydropyridine-3-carboxy]butanedioate.

10. Procédé selon la revendication 9, dans lequel ladite hydrolyse sélective est réalisée en présence d'une base sélectionnée parmi l'hydroxyde de potassium, l'hydroxyde de sodium, l'hydroxyde de lithium, l'hydroxyde de calcium ou l'hydroxyde de césium et un solvant sélectionné parmi l'éthylène glycol, le 1,3-propanediol, le 1,4-butanediol, le tert-butanol, le n-butanol, l'alcool isopropylique, l'éthanol, le méthanol, le n-propanol, le n-pentanol, l'alcool isoamylique, l'acétone, le tétrahydrofurane, le diméthoxyéthane, le polyéthylène glycol ou l'eau.

11. Procédé selon la revendication 6 ou la revendication 10, dans lequel ledit acide (R)-5-(méthoxycarbonyl)-2,6-diméthyl-4-(3-nitrophényl)-1,4-dihydropyridine-3-carboxylique est converti en barnidipine ou en un sel pharmaceutiquement acceptable de celle-ci, comprenant les étapes de
a) couplage de l'acide (R)-5-(méthoxycarbonyl)-2,6-diméthyl-4-(3-nitrophényl)-1,4-dihydropyridine-3-carboxylique avec de la (S)-1-benzyl-3-hydroxypyrrolidine en présence d'un agent d'activation acide sélectionné parmi le chlorure de thionyle, le pentachlorure de phosphore, le chlorure d'oxalyle, le chlorure de pivaloyle, le N,N'-dicyclohexylcarbodiimide (DCC), le 1,1'-carbonyldiimidazole (CDI) ou la 3-(éthyliminométhyléneamino)-N,N-diméthylpropan-1-aminé (EDC) pour obtenir la barnidipine ;
b) conversion éventuelle de la barnidipine en un sel pharmaceutiquement acceptable de celle-ci.

12. Procédé selon la revendication 11, dans lequel ladite barnidipine obtenue à l'étape a) est purifiée par un procédé comprenant les étapes de
a) traitement de la barnidipine avec un agent de formation de sel pour obtenir un sel d'addition d'acide de barnidipine ;
b) purification dudit sel d'addition d'acide de barnidipine ; et
c) conversion du sel d'addition d'acide de barnidipine pur en barnidipine, dans lequel ledit agent de formation de sel est un agent de séparation chirale.

13. Procédé selon la revendication 12, dans lequel ledit agent de séparation chirale est sélectionné parmi l'acide L-malique, l'acide D-malique, l'acide L-tartrique, l'acide D-tartrique, l'acide dibenzoyl-L-tartrique, l'acide dibenzoyl-D-tartrique, l'acide di-p-toluoyl-L-tartrique ou l'acide di-p-toluoyl-D-tartrique.

14. Procédé de préparation de l'acide (R)-5-(méthoxycarbonyl)-2,6-diméthyl-4-(3-nitrophényl)-1,4-dihydropyridine-3-carboxylique comprenant les étapes de
a) fourniture d'un composé chiral de formule II ou de formule III 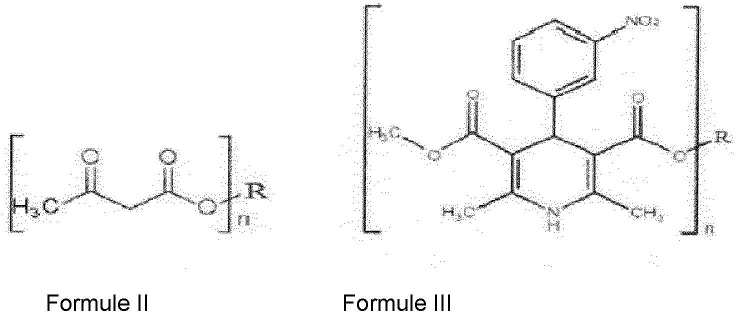 où R est un fragment auxiliaire chiral ; et n = 1 à 4 ; et
b) conversion dudit composé chiral de formule II ou de formule III en acide (R)-5-(méthoxycarbonyl)-2,6-diméthyl-4-(3-nitrophényl)-1,4-dihydropyridine-3-carboxylique, dans lequel ledit fragment auxiliaire chiral est sélectionné dans le groupe constitué par le butanedioate (2S,3S)-disubstitué, le (1R,2S,5R)-2-isopropyl-5-méthyl-cyclohexyle, le N-benzyl-3(S)-pyrrolidinyle, l'ester de l'acide phénylacétique (S)-alpha-substitué et le butanedioate (S)-alpha-substitué.

15. Procédé selon la revendication 14, dans lequel ledit acide (R)-5-(méthoxycarbonyl)-2,6-diméthyl-4-(3-nitrophényl)-1,4-dihydropyridine-3-carboxylique est converti en barnidipine ou en un sel pharmaceutiquement acceptable de celle-ci.

16. Composé chiral de formule III 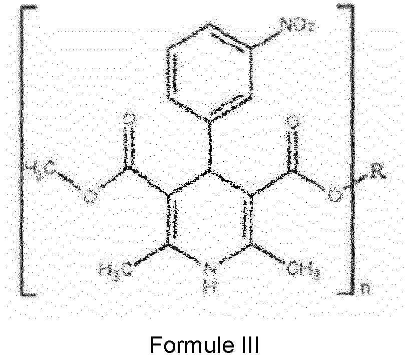 où R est un fragment auxiliaire chiral sélectionné dans le groupe constitué par le butanedioate (2S,3S)-disubstitué, le (1R,2S,5R)-2-isopropyl-5-méthyl-cyclohexyle, l'ester de l'acide phénylacétique (S)-alpha-substitué et le butanedioate (S)-alpha-substitué ; et n = 1 ou 2.

17. Composé selon la revendication 16, dans lequel ledit composé est sélectionné parmi la formule III A, III B, III D ou III E, 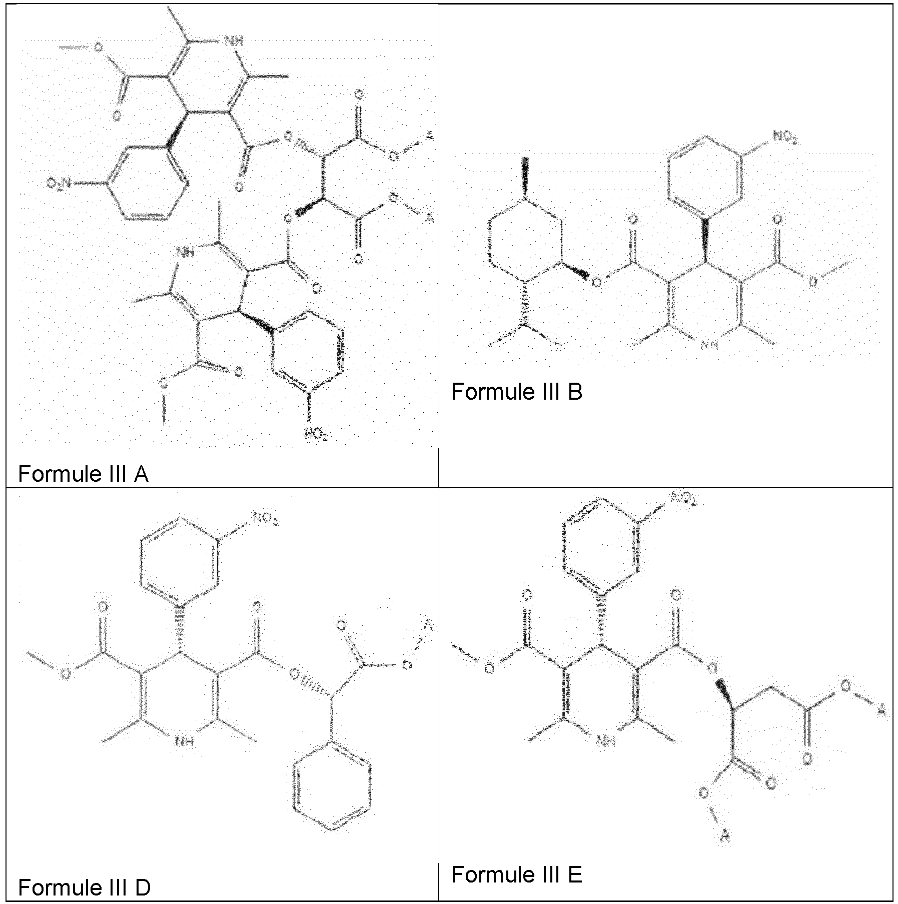 où A est sélectionné parmi un groupe méthyle, éthyle, n-propyle, isopropyle ou benzyle.

18. Utilisation d'un composé de formule II et/ou de formule III dans la préparation de barnidipine ou d'un sel pharmaceutiquement acceptable de celle-ci.

19. Utilisation selon la revendication 18, dans laquelle le composé de formule III est soumis à une hydrolyse sélective en présence d'éthylène glycol de sorte à préparer de la barnidipine ou un sel pharmaceutiquement acceptable de celle-ci.
